# EUROPEAN PATENT APPLICATION

(11) **EP 4 847 526 A1**
(43) Date of publication of application: **02.12.2026**
(21) Application number: 25744618.7
(22) Date of filing: 22.01.2025
(51) Int. Cl.: C07K 16/18, C12N 15/13, C07K 16/46, C07K 19/00, C12N 5/10, C12N 15/63, A61K 47/68, A61K 39/395

(54) **ANTI-ALBUMIN ANTIBODY OR ANTIGEN-BINDING FRAGMENT THEREOF AND USE THEREOF**

(30) Priority: 22.01.2024 CN 202410088901
(71) Applicant: Quaerite Biopharm Research (Beijing) Co., Ltd., Beijing 100195 (CN)
(72) Inventor: HUANG, Jinliang, Beijing 100195 (CN); LIU, Huiqin, Beijing 100195 (CN); YE, Junxiao, Beijing 100195 (CN)
(74) Representative: HGF
(86) International application number: PCT/CN2025/073982
(87) International publication number: WO 2025/157180

(57) **Abstract**

Provided are an anti-albumin antibody or an antigen-binding fragment thereof and the use thereof, and an anti-albumin nanobody or an antigen-binding fragment thereof with improved affinity. The anti-albumin antibodies or antigen-binding fragments thereof can bind to albumins from different species with high affinity. In addition, the anti-albumin antibody can be linked to a bioactive effector molecule to form a fusion construct without affecting the activity of the bioactive effector molecule.

## Description

### TECHNICAL FIELD

The present disclosure relates to the technical field of biomedicine, and particularly to an anti-albumin antibody or an antigen-binding fragment thereof and use thereof.

### BACKGROUND

Albumin is the most abundant protein in plasma (with a concentration of about 40 mg/mL), which is essential for maintaining plasma osmotic pressure. Also, albumin is an important transport vehicle for endogenous ligands (such as fatty acids, metal ions, hormones, etc.) and exogenous ligands (such as drugs).

Albumin has a molecular weight of 66.5 kDa and is able to bind to the neonatal Fc receptor (FcRn) on the cell surface under weakly acidic conditions. However, its binding capacity is weak under neutral conditions. Therefore, after albumin binds to FcRn, it is endocytosed into the endosome. With the acidification of the endosomal environment, the binding capacity of albumin to FcRn is enhanced, and albumin is brought back to the cell surface by FcRn or is transported across cells. Therefore, albumin can achieve a long half-life by relying on the recycling mechanism of FcRn. The half-life of human serum albumin is about 3 weeks. Therefore, small molecule compounds that bind to albumin or proteins that are expressed in fusion with albumin (such as the marketed drugs Tanzeum^{®} and Idelvion^{®}) have significantly improved pharmacokinetic properties, resulting in a longer half-life, which can potentially extend the dosing interval and provide patients with a better medication regimen.

However, due to the differences in the sequences of albumin in different species, the affinity of albumin of different species for FcRn in its corresponding species is also different. Therefore, for albumin-bound small molecule drugs or albumin fusion proteins, the pharmacokinetic evaluation and pharmacodynamic evaluation in small animals may not necessarily reflect the effect in humans. Therefore, the sequence differences of albumin in different species bring great challenges to the translational research of drugs in which albumin serves as a carrier or albumin fusion protein drugs.

Therefore, the development of antibodies that cross-bind to albumin from multiple species can potentially solve this problem. Since anti-albumin antibodies can bind to albumin of different species with similar affinity, by conjugating small molecule compounds with anti-albumin antibodies, or fusing antibodies that bind to specific antigens with anti-albumin antibodies and expressing, the pharmacokinetic and pharmacodynamic studies of this drug molecule in small animals (such as mice and rats) can provide reliable reference value for the pharmacokinetic and pharmacodynamic performance of future drugs in humans. A single domain antibody or a nanobody is a variable region domain of a heavy-chain antibody that naturally lacks light chains in camelids, which has a simple structure, and it is a minimal antibody unit with complete antigen-binding activity. Nanobodies have a molecular weight of about 13 kDa and are characterized by ease of engineering, high thermal stability, and good water solubility.

In order to predict the future effect of an active molecule in humans, its behavior in animals is usually validated in relevant animal models, such as analysis of the efficacy, pharmacokinetics, drug distribution, and drug metabolism of the active molecule. Rodents, such as mice and rats, are commonly used laboratory animal models due to their small individual size, short reproductive cycle, similar genetic background among individuals, and low experimental costs. This requires that the active molecule has the characteristics of binding to human, mouse, and rat targets. For an anti-albumin single domain antibody or nanobody, it is required that the antibody can bind to multiple species of albumin, such as human, mouse, and rat, so as to facilitate *in vivo* animal evaluation experiments of the anti-albumin nanobody and its fusion construct in rodents, such as mice and rats.

### SUMMARY

To overcome the defects of the prior art, provided in the present application are an anti-albumin antibody or an antigen-binding fragment thereof and use thereof, and an anti-albumin antibody or an antigen-binding fragment thereof with improved affinity. The anti-albumin antibody or the antigen-binding fragment thereof can bind to albumin from different species with high affinity. In addition, the anti-albumin antibody can be linked to a bioactive effector molecule to form a fusion construct without affecting the activity of the bioactive effector molecule. Specifically:
In a first aspect of the present disclosure, provided is an anti-albumin antibody or an antigen-binding fragment thereof, comprising CDR-H1, CDR-H2, and/or CDR-H3 of a heavy chain variable region.
An amino acid sequence of CDR-H1 comprises SEQ ID NO: 1 or 58, or an amino acid sequence having at least 80% identity to the amino acid sequence set forth in SEQ ID NO: 1 or 58;
an amino acid sequence of CDR-H2 comprises any one of SEQ ID NOs: 3-4, 6, and 40, or an amino acid sequence having at least 80% identity to the amino acid sequence set forth in any one of SEQ ID NOs: 3-4, 6, and 40;
an amino acid sequence of CDR-H3 comprises any one of SEQ ID NOs: 7-8 and 59, or an amino acid sequence having at least 80% identity to the amino acid sequence set forth in any one of SEQ ID NOs: 7-8 and 59.
X₁₋₁₀ in SEQ ID NO: 58 (X₁YYMS), SEQ ID NO: 40 (GISVX₂X₃X₄X₅LDYADAVX₆G), and SEQ ID NO: 59 (ASGP X₇X₈LRX₉X₁₀AP) may be any natural amino acid residue, such as alanine (A), arginine (R), asparagine (N), aspartic acid (D), cysteine (C), glutamine (Q), glutamic acid (E), glycine (G), histidine (H), isoleucine (I), leucine (L), lysine (K), methionine (M), phenylalanine (F), threonine (T), proline (P), serine (S), tryptophan (W), tyrosine (Y), or valine (V).
In one specific embodiment of the present disclosure, X₁ in SEQ ID NO: 58 (X₁YYMS) represents N or E;
X₂X₃ in SEQ ID NO: 40 (GISVX₂X₃X₄X₅LDYADAVX₆G) represents DS, DA, EG, DM, or DG, X₄X₅ represents SF or WY, and X₆ represents K, A, R, or H; preferably,
X₂X₃ represents DG, X₄X₅ represents SF, and X₆ represents K (SEQ ID NO: 5);
X₂X₃ represents DA, X₄X₅ represents SF, and X₆ represents K (SEQ ID NO: 20);
X₂X₃ represents DS, X₄X₅ represents SF, and X₆ represents K (SEQ ID NO: 21);
X₂X₃ represents EG, X₄X₅ represents SF, and X₆ represents K (SEQ ID NO: 22);
X₂X₃ represents DG, X₄X₅ represents SF, and X₆ represents A (SEQ ID NO: 31);
X₂X₃ represents DG, X₄X₅ represents SF, and X₆ represents R (SEQ ID NO: 32);
X₂X₃ represents DG, X₄X₅ represents SF, and X₆ represents H (SEQ ID NO: 33);
X₂X₃ represents DG, X₄X₅ represents WY, and X₆ represents K (SEQ ID NO: 50); or
X₂X₃ represents DM, X₄X₅ represents SF, and X₆ represents K (SEQ ID NO: 51).
X₇X₈ in SEQ ID NO: 59 (ASGPX₇X₈LRX₉X₁₀AP) represents QG, IW, LW, or VG, and X₉X₁₀ represents LG or WW. Preferably,
X₇X₈ represents QG, and X₉X₁₀ represents LG (SEQ ID NO: 9);
X₇X₈ represents IW, and X₉X₁₀ represents LG (SEQ ID NO: 52);
X₇X₈ represents LW, and X₉X₁₀ represents LG (SEQ ID NO: 53);
X₇X₈ represents QG, and X₉X₁₀ represents WW (SEQ ID NO: 54); or
X₇X₈ represents VG, and X₉X₁₀ represents LG (SEQ ID NO: 55).

In one specific embodiment of the present disclosure, the amino acid sequences of the CDR-H1, the CDR-H2, and the CDR-H3 comprise any one of the following groups (see Table 1 for details):
A) SEQ ID NO: 1, 3, 7;
B) SEQ ID NO: 1, 4, 8;
C) SEQ ID NO: 2, 5, 9;
D) SEQ ID NO: 2, 6, 9;
E) SEQ ID NO: 2, 20, 9;
F) SEQ ID NO: 2, 21, 9;
G) SEQ ID NO: 2, 22, 9;
H) SEQ ID NO: 2, 31, 9;
I) SEQ ID NO: 2, 32, 9;
J) SEQ ID NO: 2, 33, 9;
K) SEQ ID NO: 2, 5, 52;
L) SEQ ID NO: 2, 50, 53;
M) SEQ ID NO: 49, 5, 53;
N) SEQ ID NO: 49, 5, 54;
O) SEQ ID NO: 2, 51, 55.

**Table 1. Amino acid sequences of CDR-H1, CDR-H2, and CDR-H3 of candidate antibodies**

| Candidate antibody number | SEQ ID NO: x | CDR-H1 | SEQ ID NO: x | CDR-H2 | SEQ ID NO: x | CDR-H3 |
|---|---|---|---|---|---|---|
| 2049 | 1 | YYAIG | 3 | CINSSGYSTNYANSVKG | 7 | AALECRTYEYDY |
| 3004 | 1 | YYAIG | 4 | CISSSGGSTNYADSVKG | 8 | ALLECRRDYRYL EG |
| 3005 | 2 | NYYMS | 5 | GISVDGSFLDYADAVKG | 9 | ASGPQGLRLGAP |
| 3007 | 2 | NYYMS | 6 | GISIDSSILDYTDAVKG | 9 | ASGPQGLRLGAP |
| 3005Hz6 (DA) | 2 | NYYMS | 20 | GISVDASFLDYADAVKG | 9 | ASGPQGLRLGAP |
| 3005Hz6 (DS) | 2 | NYYMS | 21 | GISVDSSFLDYADAVKG | 9 | ASGPQGLRLGAP |
| 3005Hz6 (EG) | 2 | NYYMS | 22 | GISVEGSFLDYADAVKG | 9 | ASGPQGLRLGAP |
| 3005 K65A | 2 | NYYMS | 31 | GISVDGSFLDYADAVAG | 9 | ASGPQGLRLGAP |
| 3005 K65R | 2 | NYYMS | 32 | GISVDGSFLDYADAVRG | 9 | ASGPQGLRLGAP |
| 3005 K65H | 2 | NYYMS | 33 | GISVDGSFLDYADAVHG | 9 | ASGPQGLRLGAP |
| 1004 | 2 | NYYMS | 5 | GISVDGSFLDYADAVKG | 52 | ASGPIWLRLGAP |
| 1012 | 2 | NYYMS | 50 | GISVDGWYLDYADAVKG | 53 | ASGPLWLRLGAP |
| 1036 | 49 | EYYMS | 5 | GISVDGSFLDYADAVKG | 53 | ASGPLWLRLGAP |
| 1068 | 49 | EYYMS | 5 | GISVDGSFLDYADAVKG | 54 | ASGPQGLRWWA P |
| 2028 | 2 | NYYMS | 51 | GISVDMSFLDYADAVKG | 55 | ASGPVGLRLGAP |

Preferably, the amino acid sequences of the CDR-H1, the CDR-H2, and the CDR-H3 are arranged in the order from the N terminus to the C terminus. The amino acids in the CDRs of the antibody in the present application are defined by using the Kabat numbering system. The anti-albumin antibody or the antigen-binding fragment thereof comprises a heavy chain variable region.

Preferably, the anti-albumin antibody or the antigen-binding fragment thereof comprises a humanized sequence, wherein an engineered site of the humanized sequence is located in a non-CDR region, and further preferably, the engineered site of the humanized sequence is located in a framework region and/or a constant region of the antibody.

Preferably, the structure of the anti-albumin antibody or the antigen-binding fragment thereof comprises a nanobody, a chimeric antibody, a Fab fragment, a Fab' fragment, an Fd fragment, an Fv fragment, a dAb fragment, a F(ab')₂ fragment, a single chain antibody (single chain antibody fragment, scFv), or a linear antibody.

The anti-albumin antibody or the antigen-binding fragment thereof may be a single domain antibody or a nanobody.

In one specific embodiment of the present disclosure, the anti-albumin antibody or the antigen-binding fragment thereof is a nanobody, which, at the same mass, has a higher molar concentration and can bind to more antigen molecules as compared to a full-length IgG antibody, Fab, or scFv.

The anti-albumin antibody or the antigen-binding fragment thereof may bind to mammalian albumin.

Preferably, the mammal includes a human or a non-human mammal, and the non-human mammal may be a wild animal, a zoo animal, an economic animal, a companion animal, a laboratory animal, or the like. Preferably, the non-human mammal includes, but is not limited to, a pig, a cow, a sheep, a horse, a donkey, a fox, a raccoon dog, a mink, a camel, a dog, a cat, a rabbit, a rodent (e.g., a rat, a mouse, a guinea pig, a hamster, a gerbil, a chinchilla, and a squirrel), a monkey, etc.

Preferably, an amino acid sequence of the anti-albumin antibody or the antigen-binding fragment thereof comprises any one of the amino acid sequences of SEQ ID NOs: 10-13, 16-19, 23-25, 34-36, and 44-48, or has at least 80% identity to any one of the amino acid sequences of SEQ ID NOs: 10-13, 16-19, 23-25, 34-36, and 44-48.

In one specific embodiment of the present disclosure, the amino acid sequence of the anti-albumin antibody or the antigen-binding fragment thereof is set forth in any one of the amino acid sequences of SEQ ID NOs: 10-13, 16-19, 23-25, 34-36, and 44-48.

The anti-albumin antibody or the antigen-binding fragment thereof can be constructed using any conventional method in the prior art, such as artificial synthesis or eukaryotic expression or prokaryotic expression.

In a second aspect of the present disclosure, provided is use of the anti-albumin antibody or the antigen-binding fragment thereof described above, wherein the use comprises:
A. use in preparing a fusion construct comprising the anti-albumin antibody or the antigen-binding fragment thereof described above and a bioactive effector molecule linked to the anti-albumin antibody or the antigen-binding fragment thereof;
B. use in screening for a bioactive effector molecule, wherein a candidate bioactive effector molecule is linked to the anti-albumin antibody or the antigen-binding fragment thereof, and the biological activity of the candidate bioactive effector molecule is detected; or
C. use in detecting albumin.

In a third aspect of the present disclosure, provided is a fusion construct comprising one or more of the anti-albumin antibody or the antigen-binding fragment thereof described above and/or a bioactive effector molecule linked to the anti-albumin antibody or the antigen-binding fragment thereof.

Preferably, the fusion construct comprises multiple anti-albumin antibodies or antigen-binding fragments thereof, which may be the same or different.

Preferably, the fusion construct may also comprise multiple bioactive effector molecules, which may be the same or different. Preferably, the bioactive effector molecule includes, but is not limited to, a small molecule compound or a macromolecular compound.

Preferably, the small molecule compound includes, but is not limited to, any small molecule drug, such as antitumor drugs, protein kinase inhibitors, antiviral drugs, antibiotics, anti-Alzheimer drugs, anti-Parkinson drugs, anti-inflammatory drugs, anti-allergic drugs, antihypertensive drugs, anti-thromboembolic drugs, antiepileptic drugs, antidepressants, stroke drugs, autism drugs, or the like.

Preferably, the small molecule drug includes, but is not limited to, one or more of donepezil, rasagiline, entacapone, rotigotine, camptothecin, paclitaxel, sunitinib, sorafenib, warfarin, curcumin, methotrexate, docetaxel, carbamazepine, Seroxat, olanzapine, glyburide, nimodipine, idebenone, sulpiride, or deruxtecan.

In one specific embodiment, the small molecule drug is deruxtecan.

Preferably, the macromolecular compound includes, but is not limited to, an antibody, a ligand that activates or inhibits a receptor, a ligand of a protein, a bioactive enzyme, a nucleic acid medicament, or a combination thereof.

Preferably, the antibody in the macromolecular compound comprises an antibody or an antigen-binding fragment thereof to other targets different from albumin.

Further preferably, the structure of the antibody or the antigen-binding fragment thereof to the other targets includes one of or a combination of two or more of a nanobody, a chimeric antibody, a Fab fragment, a Fab' fragment, an Fd fragment, an Fv fragment, a dAb fragment, a F(ab')₂ fragment, a single chain antibody (single chain antibody fragment, scFv), or a linear antibody.

Preferably, the other targets are selected from vascular endothelial growth factor A (VEGFA), vascular endothelial growth factor B (VEGFB), vascular endothelial growth factor receptor (VEGFR), fibroblast growth factor (FGF), fibroblast growth factor receptor (FGFR), placental growth factor (PIGF), platelet-derived growth factor (PDGF), transforming growth factor (TGF), integrin, integrin receptor, interleukin (such as IL-1β, IL-2, IL-3, IL-4, IL-10, IL-12, IL-15, IL-17, IL-23, etc.), interleukin receptor (such as IL1R1, IL2Rα, IL3R, IL4Rα, IL10R, IL12R, IL15Rα, IL17R, IL23R, etc.), proprotein convertase subtilisin/kexin type 9 (PCSK9), tumor necrosis factor α (TNFα), tumor necrosis factor receptor (TNFR), receptor activator of nuclear factor-κB ligand (RANKL), G protein-coupled receptor (GPCR), glucagon-like peptide-1 receptor (GLP1R), cluster of differentiation 3 (CD3), cluster of differentiation 105 (CD105), cluster of differentiation 19 (CD19), cluster of differentiation 20 (CD20), cluster of differentiation 22 (CD22), cluster of differentiation 25 (CD25), cluster of differentiation 27 (CD27), cluster of differentiation 28 (CD28), cluster of differentiation 30 (CD30), cluster of differentiation 33 (CD33), cluster of differentiation 38 (CD38), cluster of differentiation 40 (CD40), cluster of differentiation 47 (CD47), cluster of differentiation 80 (CD80/B7-1), cluster of differentiation 86 (CD86/B7-2), cluster of differentiation 96 (CD96), cluster of differentiation 99 (CD99), cluster of differentiation 111 (CD111), cluster of differentiation 112 (CD112), cluster of differentiation 123 (CD123), cluster of differentiation 133 (CD133), cluster of differentiation 138 (CD138), cluster of differentiation 155 (CD155), cluster of differentiation 171 (CD171), Claudin 18.2 (CLDN18.2), TNF receptor superfamily member 4 (tumor necrosis factor receptor superfamily, member 4, TNFRSF4/OX40/CD134), inducible T-cell co stimulator (ICOS), cytotoxic T-lymphocyte-associated protein 4 (CTLA4), TNF receptor superfamily member 9 (tumor necrosis factor receptor superfamily, member 9, TNFRSF9/4-1BB/CD137), T cell antigen receptor (T cell receptor, TCR), B- and T-lymphocyte attenuator (BTLA), T lymphocyte immunoglobulin mucin 3 (T cell immunoglobulin domain and mucin domain-3, TIM-3), lymphocyte activation gene 3 (LAG3), galectin-9 (GAL9), programmed cell death ligand 1 (programmed cell death 1 ligand 1, PD-L1), programmed cell death ligand 2 (programmed cell death 1 ligand 2, PD-L2), programmed death receptor 1 (programmed cell death protein 1, PD-1), T cell immunoglobulin and ITIM domain protein (T cell immune receptor with Ig and ITIM domains, TIGIT), epidermal growth factor receptor (EGFR), human epidermal growth factor receptor 2 (HER2), prostate stem cell antigen (PSCA), carcinoembryonic antigen (CEA), familial adenomatous polyposis (FAP), epidermal growth factor receptor variant type III (EGFRvIII) antibody, B cell maturation antigen (BCMA), prostate specific membrane antigen (PSMA), saccharide antigen 125 (cancer antigen 125, CA125), Ephrin A receptor 2 (EphA2), cellular-mesenchymal epithelial transition factor (c-Met), L1-cell adhesion molecule (L1CAM), human signaling lymphocytic activation molecule family member 7 (signaling lymphocytic activation molecule family, member 7, SLAMF7/CS1), receptor tyrosine kinase-like orphan receptor 1 (ROR1), recombinant Mycobacterium tuberculosis fusion protein, human esophageal squamous cell carcinoma antigen 1 (New York esophageal squamous cell carcinoma-1, NY-ESO-1), mucin 1 (MUC1), mucin 16 (MUC16), mesothelin, cluster of differentiation 174 (LewisY/CD174), glypican 3 (GPC3), disialoganglioside (disialoganglioside-GD2, GD2), eukaryotic division factor-like and proliferation-associated protein (EPG), delta-like ligand 3 (DLL3), and trophoblast glycoprotein (TPBG/5T4).

In one specific embodiment, the other targets are VEGFA.

Preferably, the anti-albumin antibody or the antigen-binding fragment thereof is directly or indirectly linked to the bioactive effector molecule.

Preferably, the indirect linkage may be a linkage via a linker, a functional domain, and/or a linker for conjugation.

The linker is selected from a linker peptide, an oligopeptide, an oligopeptide polymer, a polypeptide, a polypeptide polymer, PEG, a nucleic acid, a polysaccharide, a fatty chain, biotin, streptavidin, and avidin.

The functional domain is one of or a combination of two or more of an Fc fragment, serum albumin, a cytokine, transferrin, and a scaffold protein.

The linker for conjugation comprises a functional group linker.

The functional group linker comprises sulfhydryl, amino, hydroxy, and/or carboxyl reactive groups, and may covalently conjugate the anti-albumin antibody or the antigen-binding fragment thereof to the bioactive effector molecule.

Preferably, the bioactive effector molecule is directly or indirectly linked to the N terminus, C terminus, and/or internal residues of the anti-albumin antibody or the antigen-binding fragment thereof.

Preferably, the fusion construct comprises one or more bioactive effector molecules.

For example, in the fusion construct, from N terminus to C terminus, the linking order of the antibody or the antigen-binding fragment thereof and the bioactive effector molecule is as follows:
the anti-albumin antibody or the antigen-binding fragment thereof, a linker peptide, and the bioactive effector molecule;
the bioactive effector molecule, a linker peptide, and the anti-albumin antibody or the antigen-binding fragment thereof;
the anti-albumin antibody or the antigen-binding fragment thereof, a linker peptide, the first bioactive effector molecule, a linker peptide, and the second bioactive effector molecule; or
the first bioactive effector molecule, a linker peptide, the anti-albumin antibody or the antigen-binding fragment thereof, a linker peptide, and the second bioactive effector molecule, and the like, wherein the linkage may not include the linker peptide. In one specific embodiment, the bioactive effector molecule comprises any one of the amino acid sequences of SEQ ID NOs: 26-27, or has at least 80% identity to any one of the amino acid sequences of SEQ ID NOs: 26-27.

In one specific embodiment, the fusion construct comprises any one of the amino acid sequences of SEQ ID NOs: 28-30 and 61, or has at least 80% identity to any one of the amino acid sequences of SEQ ID NOs: 28-30 and 61.

In one specific embodiment, the fusion construct includes an Fc fragment.

Further preferably, the Fc fragment comprises SEQ ID NO: 38, or has at least 80% identity to the amino acid sequence set forth in SEQ ID NO: 38.

In one specific embodiment, the fusion construct comprises any one of the amino acid sequences of SEQ ID NOs: 14-15, or has at least 80% identity to any one of the amino acid sequences of SEQ ID NOs: 14-15.

Preferably, the fusion construct further comprises a secretion peptide. The secretion peptide is linked at the N terminus of the fusion construct. Further preferably, the amino acid sequence of the secretion peptide may be SEQ ID NO: 37 or 42.

Preferably, the fusion construct further comprises a tag.

Preferably, the tag is linked at the C terminus of any one of the antibodies or the antigen-binding fragments thereof, or the fusion construct.

Preferably, the fusion construct comprises a conjugate of the anti-albumin antibody and a drug, which comprises any of the anti-albumin antibodies or the antigen-binding fragments thereof described above, and the bioactive effector molecule is the drug. The drug is covalently bound to any of the anti-albumin antibodies or the antigen-binding fragments thereof described above.

In a fourth aspect of the present disclosure, provided is a nucleic acid encoding the anti-albumin antibody or the antigen-binding fragment thereof described above or the fusion construct described above. For example, the nucleic acid comprises DNA and/or mRNA.

In some embodiments, the nucleic acid is DNA, which encodes the antibody or the antigen-binding fragment described herein, or the fusion construct described above.

Preferably, the nucleotide sequence encoding the anti-albumin antibody or the antigen-binding fragment thereof comprises any nucleotide sequence of SEQ ID NOs: 63-75, 79-81, and 83-87, or a degenerate sequence thereof, or a nucleotide sequence having at least 80% identity to any nucleotide sequence of SEQ ID NOs: 63-75, 79-81, and 83-87 and having a function of encoding an anti-albumin antibody or an antigen-binding fragment thereof.

Preferably, the nucleotide sequence encoding the fusion construct described above comprises any nucleotide sequence of SEQ ID NOs: 76-78 and 82, or a degenerate sequence thereof, or a nucleotide sequence having at least 80% identity to any nucleotide sequence of SEQ ID NOs: 76-78 and 82 and having a function of encoding the fusion construct described above. In a fifth aspect of the present disclosure, provided is a vector comprising the nucleic acid described above.

The vector is capable of expression *in vivo* or *in vitro* or *ex vivo*. Preferably, the vector is a prokaryotic expression vector, a viral expression vector, or a eukaryotic expression vector, such as an *Escherichia coli* series vector, a phage, etc.

In a sixth aspect of the present disclosure, provided is a host cell comprising the nucleic acid described above or the vector described above.

The host cell may be a eukaryotic cell or a prokaryotic cell.

The eukaryotic cell includes animal and plant cells, such as T cells, yeast cells, HEK293 cells, 293F cells, CHO cells, etc. The prokaryotic cell includes, for example, *Escherichia coli*, etc.

In a seventh aspect of the present disclosure, provided is a preparation method for a host cell, which comprises introducing the nucleic acid or the vector described above into a host cell.

In an eighth aspect of the present disclosure, provided is a method for preparing the anti-albumin antibody or the antigen-binding fragment thereof described above or the fusion construct described above. The preparation method comprises culturing the host cell described above, and expressing the anti-albumin antibody or the antigen-binding fragment thereof or the fusion construct.

**In** a ninth aspect of the present disclosure, provided is a product for treating and/or diagnosing a disease, which comprises any one of the following:
A) the anti-albumin antibody or the antigen-binding fragment thereof described above;
B) the fusion construct described above;
C) the nucleic acid described above;
D) the vector described above; or
F) the host cell described above.

Preferably, the anti-albumin antibody or the antigen-binding fragment thereof and/or the biological effector molecule have activity for treating and/or diagnosing a disease, and further preferably, the anti-albumin antibody or the antigen-binding fragment thereof serves as a carrier in the product for treating and/or diagnosing a disease.

The disease includes, but is not limited to, tumors, infectious diseases, Alzheimer's disease, Parkinson's disease, inflammatory diseases, epilepsy, depression, stroke, autism, allergic diseases, hypertension, thrombosis, or diseases associated with signaling pathways of the other targets.

Preferably, the fusion construct is a conjugate of the anti-albumin antibody and a drug, which comprises any of the anti-albumin antibodies or the antigen-binding fragments thereof described above, and the bioactive effector molecule is the drug. The drug is covalently bound to any of the anti-albumin antibodies or the antigen-binding fragments thereof described above. In a tenth aspect of the present disclosure, provided is a method for detecting albumin, which comprises contacting a sample to be tested with the anti-albumin antibody or the antigen-binding fragment thereof described above, and subsequently detecting the content of a complex formed by albumin and the anti-albumin antibody or the antigen-binding fragment thereof. The detection method is to detect the existence or the content of albumin. The existence indicates presence or absence, and the content may be an expression level, a protein concentration, or the like. Preferably, the albumin is from a mammal, and more preferably, the mammal is from a human, a mouse, or a monkey.

In an eleventh aspect of the present disclosure, provided is a method for treating and/or preventing a disease, which comprises administering to an individual the anti-albumin antibody or the antigen-binding fragment thereof described above, the fusion construct described above, the nucleic acid described above, the vector described above, the host cell described above, or the product for treating and/or diagnosing a disease described above.

The disease includes, but is not limited to, tumors, infectious diseases, Alzheimer's disease, Parkinson's disease, inflammatory diseases, epilepsy, depression, stroke, autism, allergic diseases, hypertension, thrombosis, or diseases associated with signaling pathways of the other targets.

In one specific embodiment, the disease includes a disease associated with the VEGFA signaling pathway. Further preferably, it may be a tumor, abnormal vascular proliferation, an ophthalmic disease involving angiogenesis (e.g., a fundus vascular disease), or the like.

Preferably, the target cell is selected from cells expressing VEGFA, such as cardiomyocytes, renal proximal tubule cells, hepatocytes, vascular endothelial cells, granulosa cells, specialized epithelial cells, mesenchymal cells, macrophages, platelets, dendritic cells, activated T cells, retinal pigment epithelial cells, Muller cells in the retina, astrocytes, osteoblasts, bronchial and alveolar epithelial cells, pericytes, vascular smooth muscle cells, myofibroblasts, keratinocytes, renal mesangial cells, tumor cells, etc.

In a twelfth aspect of the present disclosure, provided is use of the anti-albumin antibody or the antigen-binding fragment thereof described above, the fusion construct described above, the nucleic acid described above, the vector described above, or the host cell described above in preparing a product for treating and/or preventing a disease, wherein the anti-albumin antibody or the antigen-binding fragment thereof and/or the bioactive effector molecule have activity for treating and/or diagnosing a disease.

Preferably, the anti-albumin antibody or the antigen-binding fragment thereof serves as a carrier in the product for treating and/or diagnosing a disease.

The disease includes, but is not limited to, tumors, infectious diseases, Alzheimer's disease, Parkinson's disease, inflammatory diseases, epilepsy, depression, stroke, autism, allergic diseases, hypertension, thrombosis, or diseases associated with signaling pathways of the other targets.

In one specific embodiment, the disease includes a disease associated with the VEGFA signaling pathway. Further preferably, it may be a tumor, abnormal vascular proliferation, an ophthalmic disease involving angiogenesis (e.g., a fundus vascular disease), or the like.

"Fusion construct" used herein defines the fusion of the anti-albumin antibody or the antigen-binding fragment thereof of the present disclosure with an additional compound. The fusion construct may comprise one or more anti-albumin antibodies or antigen-binding fragments thereof, wherein the plurality of anti-albumin antibodies or antigen-binding fragments thereof may be the same or different. The fusion construct may include one or more additional compounds, wherein the plurality of additional compounds may also be the same or different. The compound may be a protein compound or a non-protein compound. Where the compound is a protein compound or the fusion construct comprises only a plurality of anti-albumin antibodies or antigen-binding fragments thereof, the fusion construct may also be referred to as a fusion protein. Where the compound is fused to an anti-albumin antibody or an antigen-binding fragment thereof in a conjugated form, the fusion construct may also be referred to as a conjugate.

The product described herein may be a kit or a medicament or a chip or an antibody-drug conjugate, or the like.

The "medicament" described in the present disclosure may be used for treating a human or non-human animal, such as a non-human mammal. The medicament may comprise pharmaceutically acceptable carriers, auxiliary materials, or salts common in the prior art. The medicament may be administered by any suitable route of administration, such as gastrointestinal routes (e.g., oral administration) or parenteral routes (e.g., intravenous, intramuscular, subcutaneous, intradermal, intraorgan, intranasal, intraocular, instillation, intracerebral, intrathecal, transdermal, intrarectal administrations, etc.). The medicament may be in any suitable dosage form, such as dosage forms for gastrointestinal or parenteral administration. The dosage forms preferably include, but are not limited to, tablets, pills, powders, granules, capsules, lozenges, syrups, liquids, emulsions, microemulsions, suspensions, injections, sprays, aerosols, powder aerosols, lotions, ointments, plasters, pastes, patches, eye drops, nose drops, sublingual tablets, suppositories, aerosols, effervescent tablets, dripping pills, gels, and the like. The various dosage forms of the medicament can be prepared according to conventional production methods in the field of pharmaceuticals. The medicament may comprise the anti-albumin antibody or the antigen-binding fragment thereof, the fusion construct, the nucleic acid, the vector, the host cell, the immune cell, and the like in a weight ratio of 0.01%-99.5% (specifically, 0.01%, 0.1%, 0.5%, 1%, 2%, 3%, 4%, 5%, 6%, 7%, 8%, 9%, 10%, 20%, 30%, 40%, 50%, 60%, 70%, 80%, 90%, 95%, 99%, or 99.5%). The medicament may be prepared as a reagent with a protein concentration of 1-300 mg/mL (e.g., 1 mg/mL, 5 mg/mL, 10 mg/mL, 15 mg/mL, 20 mg/mL, 25 mg/mL, 30 mg/mL, 35 mg/mL, 40 mg/mL, 45 mg/mL, 50 mg/mL, 55 mg/mL, 60 mg/mL, 65 mg/mL, 70 mg/mL, 75 mg/mL, 80 mg/mL, 85 mg/mL, 90 mg/mL, 95 mg/mL, 100 mg/mL, 105 mg/mL, 110 mg/mL, 115 mg/mL, 120 mg/mL, 130 mg/mL, 140 mg/mL, 150 mg/mL, 160 mg/mL, 170 mg/mL, 180 mg/mL, 190 mg/mL, 200 mg/mL, 210 mg/mL, 220 mg/mL, 230 mg/mL, 240 mg/mL, 250 mg/mL, 260 mg/mL, 270 mg/mL, 280 mg/mL, 290 mg/mL, or 300 mg/mL). A single administration dose of the medicament may be 0.1-1000 mg, e.g., 0.1 mg, 0.2 mg, 0.5 mg, 0.75 mg, 1 mg, 1.25 mg, 1.5 mg, 1.75 mg, 2 mg, 2.25 mg, 2.5 mg, 3 mg, 5 mg, 10 mg, 20 mg, 50 mg, 80 mg, 100 mg, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 950 mg, or 1000 mg.

The "pharmaceutically acceptable" described in the present disclosure means that it neither significantly stimulates an organism nor inhibits the biological activity and properties of the active substance in the administered product.

The "method for..." described in the present disclosure can be used for the purposes of diagnosing and treating diseases or non-disease conditions.

The "antigen-binding fragment" described in the present disclosure is a portion of an antibody that retains the specific binding activity of the antibody, i.e., any portion of the antibody that is capable of specifically binding to an epitope on a target molecule of the antibody. It includes, for example, Fab, Fab', F(ab')₂, Fv, Fd, and variants of these fragments, such as the heavy chain and/or light chain of the antibody, the heavy chain variable region and/or light chain variable region of the antibody, or a single or 2 or more CDRs from the heavy or light chains of the antibody. Among them,

a nanobody or a single domain antibody means that the variable region of the heavy chain (variable domain of heavy chain, VHH) of the antibody has independent antigen-binding activity.

A chimeric antibody refers to an antibody in which a portion of the heavy chain and/or the light chain is derived from a particular source or species, and the remainder of the heavy chain and/or the light chain is derived from different sources or species.

A single chain antibody refers to an antibody in which the variable regions of heavy and light chains are linked by a linker peptide (linker).

Fab is a monovalent fragment consisting of VL, VH, CL, and CH1 domains.

Fab' is a Fab fragment with one or more cysteine residues at the C terminus of the CH1 domain.

F(ab')₂ is a bivalent fragment comprising two Fab fragments linked by disulfide bonds at the hinge region.

Fd is an Fd fragment consisting of VH and CH1 domains.

Fv is an Fv fragment consisting of VL and VH domains of a single arm of an antibody.

A dAb fragment is an antibody fragment consisting of a VH domain.

The "linear antibody" described in the present disclosure includes one or more pairs of antibody fragments in series, which may be Fd-segments (VH-CH1), single chain antibodies (scFv), antibody fragments (Fab), or single domain antibodies (VHH).

The fragments are linked together by a linker peptide to form a continuous antibody structure.

VH represents a heavy chain variable region, VL represents a light chain variable region, CL represents a light chain constant region, and CH represents a heavy chain constant region.

The "Fc" region described in the present disclosure comprises two heavy chain fragments containing the CH2 and CH3 domains of the antibody. The two heavy chain fragments form a dimer through two or more disulfide bonds in the hinge region and are held together by the hydrophobic interactions in the CH3 domains.

The "comprises" or "comprising" described in the present disclosure is an open-ended expression. When the term is used to describe a sequence of a protein or nucleic acid, the protein or the nucleic acid may consist of the sequence, or may have additional amino acids or nucleotides at one or both ends of the protein or the nucleic acid, while still retaining the same or similar activity as the original sequence.

The "homology" or "identity" described in the present disclosure means that in the context of using an protein sequence or a nucleotide sequence, those skilled in the art can adjust the sequence according to as necessary for practice, so as to obtain a sequence having (including but not limited to) 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 70%, 80%, 81%, 82%, 83%, 84%, 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% homology to the sequence obtained by the prior art.

The "humanized antibody" described in the present disclosure refers to an antibody in which the framework regions and/or constant regions (e.g., CH regions), or the entirety or a portion of the antibody, are encoded by human antibody genes. In one specific embodiment of the present disclosure, the CDRs of the antibody are not subjected to humanization engineering.

The "individual" described in the present disclosure may be a human or a non-human mammal, and the non-human mammal may be a wild animal, a zoo animal, an economic animal, a companion animal, a laboratory animal, or the like. Preferably, the non-human mammal includes, but is not limited to, a pig, a cow, a sheep, a horse, a donkey, a fox, a raccoon dog, a mink, a camel, a dog, a cat, a rabbit, a rodent (e.g., a rat, a mouse, a guinea pig, a hamster, a gerbil, a chinchilla, and a squirrel), a monkey, etc.

The "treating" or "treatment" described in the present disclosure refers to slowing, interrupting, arresting, controlling, stopping, reducing, or reversing the progression or severity of a sign, symptom, disorder, condition, or disease after the disease has begun to progress, but does not necessarily involve the complete elimination of all disease-related signs, symptoms, conditions, or disorders.

The "preventing" described in the present disclosure refers to the practice of preventing or delaying the onset of a disease or condition or symptom in the body.

The "diagnosing" described in the present disclosure refers to the determination of whether a patient has suffered from, is suffering from, or will suffer from a disease or condition in the past, at the time of diagnosis, or in the future, or the determination of the progression or likely progression in the future of a disease.

The "tumor" described in the present disclosure may be any adverse cell proliferation (or any disease that manifests itself as adverse cell proliferation) or neoplasm, or increased predisposition or risk for adverse cell proliferation, neoplasm, or a tumor. The tumor may be benign or malignant, and may also be primary or secondary (metastatic). The neoplasm may be any abnormal growth or proliferation of cells and may be located in any tissue. Examples of the tissue include adrenal gland, adrenal medulla, anus, appendix, bladder, blood, bone, bone marrow, brain, mammary gland, cecum, central nervous system (including or excluding brain), cerebellum, cervix, colon, duodenum, endometrium, epithelial cells (e.g., renal epithelial cells), gallbladder, esophagus, glial cells, heart, ileum, jejunum, kidney, lacrimal gland, larynx, liver, lung, lymph, lymph nodes, lymphoblasts, maxilla, mediastinum, mesenterium, myometrium, nasopharynx, omentum, oral cavity, ovary, pancreas, parotid gland, peripheral nervous system, peritoneum, pleura, prostate, salivary glands, sigmoid colon, skin, small intestine, soft tissue, spleen, stomach, testis, thymus, thyroid, tongue, tonsils, trachea, uterus, vulva, and white blood cells. Further preferably, the tumor is selected from prostate cancer, breast cancer, liver cancer, glioma (e.g., neuroglioma), intestinal cancer, cervical cancer, non-small cell lung cancer, lung cancer, pancreatic cancer, gastric cancer, bladder cancer, skin cancer, rhabdomyosarcoma, tongue squamous cell carcinoma, nasopharyngeal cancer, ovarian cancer, placental choriocarcinoma, lymphoma (e.g., non-Hodgkin lymphoma, Hodgkin lymphoma, and cutaneous T-cell lymphoma), leukemia, rectal adenocarcinoma, medulloblastoma, meningioma, neurofibroma (e.g., neurofibrosarcoma), ependymoma, schwannoma, astrocytoma, melanoma, mesothelioma, myeloma, chronic myeloid leukemia, acute myelogenous leukemia, myelodysplastic syndrome, chronic lymphocytic leukemia, epidermoid cancer, colon cancer, thymus cancer, blood cancer, head and neck cancer, and oropharyngeal cancer.

The "fundus vascular disease" described in the present disclosure refers to the general term for diseases occurring in the retinal arteries or veins, including but not limited to age-related macular degeneration (AMD), diabetic macular edema (DME), diabetic retinopathy, central retinal vein occlusion, pathological myopia, neovascular glaucoma, and the like.

Antibodies or antigen-binding fragments thereof that bind to albumin with high affinity are obtained in the present disclosure. The anti-albumin nanobodies have the following advantages:
1. The obtained anti-albumin nanobodies can bind to different species of albumin with high affinity, including human, monkey, mouse, rat, and other species. Also, the humanization engineering has been completed, and the humanized anti-albumin antibodies can bind to human albumin and mouse albumin, and can bind to rat albumin. Mice and rats are commonly used laboratory animals, which makes the anti-albumin nanobody of the present disclosure have a broader application prospect.
2. Antibodies with 3.7-fold or 35.7-fold increased affinity to human albumin are obtained by performing affinity increase modification of the preferred humanized anti-albumin antibody.
3. Albumin has the characteristics of enrichment at inflammation and tumor sites. Humanized anti-albumin nanobodies can be used to prepare drugs for the treatment of immune diseases, tumors, and other diseases. By fusing anti-albumin nanobodies to other antibodies, the obtained fusion constructs can bind to albumin *in vivo,* with similar characteristics of enrichment at inflammation and tumor sites to albumin.

### BRIEF DESCRIPTION OF THE DRAWINGS

Hereinafter, examples of the present disclosure will be described in detail with reference to the drawings, where:
FIG. 1 shows the Coomassie-stained gel images of His-tag-containing antibodies Alb Nb, 2049, 3004, 3007, and 3005 after purification with Ni column, wherein (A) shows the Coomassie-stained gel images of antibodies Alb Nb, 2049, 3004, and 3007, and (B) shows the Coomassie-stained gel image of antibody 3005.
FIG. 2 shows the ELISA of fusion proteins 3005-Fc, 3007-Fc, and Alb Nb-Fc binding to albumin of different species, wherein (A) shows the HSA binding assay (HSA binding ELISA), (B) shows the MSA binding assay (MSA binding ELISA), and (C) shows the rat albumin binding assay (rat albumin binding ELISA).
FIG. 3 shows the results of the Coomassie staining after purification with Ni column of the His-tag-containing 3005 humanized antibody.
FIG. 4 shows the ELISA activity assay of antibody 3005 and a humanized antibody thereof binding to MSA.
FIG. 5 shows the ELISA activity assay of antibody 3005, its humanized antibody 3005Hz6, and control antibody Alb Nb binding to HSA and rat albumin, wherein (A) shows the HSA binding assay (HSA binding ELISA), and (B) shows the rat albumin binding assay (rat albumin binding ELISA).
FIG. 6 shows the binding and dissociation curves (SPR method) of humanized antibody 3005Hz6 to HSA and MSA, wherein (A) shows the binding and dissociation curves of antibody 3005Hz6 to HSA, and (B) shows the binding and dissociation curves of antibody 3005Hz6 to MSA, with the ordinate as the response value and the abscissa as time.
FIG. 7 shows the SDS-PAGE Coomassie-stained gel images of humanized antibody 3005Hz6 mutant proteins 3005Hz6 (EG), 3005Hz6 (DS), and 3005Hz6 (DA) after purification, wherein (A) shows the SDS-PAGE Coomassie-stained gel images of 3005Hz6 (EG) and 3005Hz6 (DS), and (B) shows the SDS-PAGE Coomassie-stained gel image of 3005Hz6 (DA).
FIG. 8 shows the ELISA activity assay (HSA binding ELISA) of humanized antibody 3005Hz6 and its mutant proteins 3005Hz6 (DA), 3005Hz6 (DS), and 3005Hz6 (EG) binding to HSA.
FIG. 9 shows the SDS-PAGE gel images of fusion proteins V1SA-3005Hz6, 3005Hz6-V1SA, and VIDP-3005Hz6 after purification with Ni column, wherein (A) shows the SDS-PAGE Coomassie-stained gel images of VISA-3005Hz6 and 3005Hz6-V1SA, and (B) shows the SDS-PAGE Coomassie-stained gel image of V1DP-3005Hz6.
FIG. 10 shows the ELISA activity assay of the fusion protein of humanized antibody 3005Hz6 and monovalent anti-VEGFA nanobody and humanized antibody 3005Hz6 binding to HSA.
FIG. 11 shows the VEGFR2 competitive ELISA activity assay of the fusion protein and monovalent nanobody V1-SA1 without pre-incubation or with pre-incubation of HSA, wherein (A) shows the results without pre-incubation of HSA, and (B) shows the results with pre-incubation of HSA.
FIG. 12 shows the flowchart of the conjugation and purification of nanobody-deruxtecan conjugate (NDC).
FIG. 13 (A) shows the structure schematic diagram of 3005 K65A-deruxtecan conjugate (NDC), and (B) shows SDS-PAGE Coomassie-stained gel images of 3005 K65A and three different 3005 K65A-deruxtecan conjugates (NDC-1, NDC-2, and NDC-3).
FIG. 14 (A) shows the ELISA activity assay (HSA binding ELISA) of 3005 K65A and 3005 K65A-deruxtecan conjugate (NDC-3) binding to HSA, and (B) shows the results of the proliferation inhibition of deruxtecan and 3005 K65A-deruxtecan conjugate (NDC-3) on Mia Paca-2 cells.
FIG. 15 shows the results of the Coomassie staining after purification of the candidate antibodies with improved affinity.
FIG. 16 shows the ELISA experiment results (HSA binding ELISA) (a method in which the antibody is coated, coat antibody) of the candidate antibodies with improved affinity binding to HSA, wherein (A) shows the ELISA experiment results at pH=7.4, and (B) shows the ELISA experiment results at pH=6.0.
FIG. 17 shows the ELISA experiment results (rat albumin binding ELISA) (a method in which the antigen is coated) of the candidate antibodies with improved affinity binding to rat albumin, wherein (A) shows the ELISA experiment results at pH=7.4, and (B) shows the ELISA experiment results at pH=6.0.
FIG. 18 shows the binding and dissociation curves (SPR method) of the antibody with improved affinity and the control antibody to HSA, wherein (A) shows the binding and dissociation curves of antibody 3005Hz6 to HSA, (B) shows the binding and dissociation curves of antibody 1068 to HSA, (C) shows the binding and dissociation curves of antibody 2028 to HSA, and (D) shows the binding and dissociation curves of the control antibody BI-V2 to HSA, with the ordinate as the response value and the abscissa as time.

### DETAILED DESCRIPTION

The technical solutions in the examples of the present disclosure will be described clearly and completely below with reference to the drawings. It is obvious that the described examples are only a part of the examples of the present disclosure, rather than all of them. Based on the examples of the present disclosure, all other examples obtained by those of ordinary skill in the art without creative work shall fall within the protection scope of the present disclosure.

### Example 1: Alpaca Immunization and Antibody Library Construction

### 1. Alpaca immunization and potency detection

Human serum albumin HSA (purchased from Sigma-Aldrich, Cat. No.: A3782) was emulsified and used to immunize alpacas. The alpaca was immunized with 1 mg of human serum albumin every time, once every 2 weeks, for a total of three times. Starting from the second immunization, serum was taken one week after immunization for potency detection.

The serum potency detection method was as follows:
HSA antigen was diluted to 1 µg/mL with CBS buffer, added to a microplate at 100 µL/well, and incubated overnight at 4 °C. The plate was washed 3 times with PBST (PBS containing 0.1% Tween 20, pH=7.4). 200 µL of skimmed milk powder was added to each well and the plate was left to stand at room temperature for 1 h, followed by plate washing with PBST 3 times. Sample addition: The immunized serum and negative serum were each diluted 1000-fold, 3000-fold, 9000-fold, 27,000-fold, 81,000-fold, and 243,000-fold. 100 µL of each dilution was added to the wells that had been blocked, and the plate was left to stand at room temperature for 1 h, followed by plate washing with PBST 3 times.

Secondary antibody: HRP-labeled Goat Anti-Alpaca IgG was added at 100 µL/well and the plate was left to stand at room temperature for 1 h, followed by plate washing with PBST 3 times.

TMB color development: TMB chromogenic solution was added to the microplate at 100 µL/well for color development for 15 min; a stopping solution was added at 50 µL/well, and the microplate was placed in a microplate reader to read the absorbance at 450 nm (OD450).

The serum potency detection results after the second and third immunizations of alpacas are shown in Table 2. It can be seen that after the serum was diluted 243,000-fold, the signal value of binding to human serum albumin was 0.59 or more, which met the library construction criteria. The alpaca serum after the third immunization was used for phage library construction.

**Table 2. Serum potency detection after the second and third albumin alpaca immunizations**

| Antigen coating | Human serum albumin HSA (1 µg/mL) | | | |
|---|---|---|---|---|
| | OD450 | | | |
| Serum dilution factor | Serum after the second immunization | Negative serum | Serum after the third immunization | Negative serum |
| 1000 | 2.1439 | 0.0734 | 2.3233 | 0.0945 |
| 3000 | 1.9308 | 0.0553 | 2.3931 | 0.054 |
| 9000 | 1.5265 | 0.0541 | 2.1041 | 0.0526 |
| 27000 | 1.3957 | 0.0442 | 1.8906 | 0.0502 |
| 81000 | 1.029 | 0.0404 | 1.4248 | 0.0407 |
| 243000 | 0.592 | 0.035 | 0.8564 | 0.035 |
| Blank well | 0.0418 | 0.0538 | 0.0073 | 0.0538 |

### 2. Construction of phage library

The peripheral blood of the alpaca was extracted. Total RNA was extracted from the isolated alpaca PBMCs using Trizol and reverse-transcribed into cDNA. The variable region VH fragments (about 700 bp in length) were amplified twice with a nanobody-specific primer. The fragments obtained above and the pcomb3X vector were digested with SfiI enzyme, and after digestion, they were mixed in an appropriate proportion and ligated using a T4 ligase. After ligation, the constructs were used for electroporation of XLl-Blue competent cells. Based on the colony growth of the dilution of competent cells on the resistant plate, the transformation library capacity of the alpaca was calculated to be 6.24 × 10⁸.

### Example 2: Antibody Library Screening to Obtain Antibodies that Cross-Bind to Human, Mouse, and Cynomolgus Monkey Albumin

### 1. Phage library screening

The screening process was as follows:
The antigens human serum albumin HSA and mouse albumin MSA (purchased from Sigma-Aldrich, Cat. No.: A3559) were coated, and a negative control (in which the microplate was coated with 3% skimmed milk powder) was set. The plate was incubated at room temperature for 1 h. The blocking buffer was discarded and washed with PBST.

About 5 × 10¹² pfu of the library was added to the antigen plate and incubated with the antigen at 37 °C for 2 h, and the plate was washed with 0.1% PBST.

Elution was performed with glycine-HCl at low pH, followed by neutralization with Tris-HCl until pH 7.4.

The eluate was mixed with *Escherichia coli* XL1-Blue, incubated at 37 °C, and shaken.

After the phage was added, the mixture was cultured at 30 °C overnight, and the supernatant was collected.

1) - 5) were repeated to obtain a second round of library.

When the second round of amplified library obtained under the condition of coated HSA was used for ELISA, the antigen HSA was coated with two coating buffers: PBS buffer or CBS buffer, followed by ELISA binding assay. The HRP-labeled Anti-M13 Antibody (purchased from Sino Biological, Cat. No.: 11973-MM05T-H) was used for the detection of the phage, which was diluted 2500-fold before use. After incubation of the detection antibody, the plate was washed, TMB color development was performed, and OD450 was read. The detection results of the second round of amplified library are shown in Table 3. It can be seen that after the second round of enrichment, a phage signal that strongly bound to HSA was generated.

**Table 3. Detection results of second round of phage library obtained by enrichment under the condition of coated HSA binding to HSA**

| Library enrichment method | Antigen coating solution for ELISA | Amplification library dilution factor | OD450 (coated with 5 µg/mL HSA) | OD450 (coated with 3% milk powder) |
|---|---|---|---|---|
| HSA coating and enrichment | CBS | 2 | 2.376 | 0.0242 |
| | | 10 | 1.9245 | 0.0358 |
| HSA coating and enrichment | PBS | 2 | 2.3696 | 0.0421 |
| | | 10 | 1.7128 | 0.0382 |

For the second round of amplified library obtained under the condition of coated MSA, antigen binding ELISA was performed. In the ELISA, CBS buffer was used to separately coat HSA and MSA. The ELISA results are shown in Table 4. It can be seen that a phage with strong binding to HSA and MSA was also obtained under this enrichment condition.

**Table 4. Detection results of second round of phage library obtained by enrichment under the condition of coated MSA binding to HSA and MSA**

| Library enrichment method | Amplification library dilution factor | OD450 (coated with 5 µg/mL HSA) | OD450 (coated with 5 µg/mL MSA) | OD450 (coated with 3% milk powder) |
|---|---|---|---|---|
| MSA coating and enrichment | 10 | 1.9721 | 1.7435 | 0.0826 |

Summary: The antigen binding ELISA of the second round of phage libraries obtained by coating HSA and MSA for enrichment was positive, indicating that phages that separately bound to HSA and MSA were enriched, and the binding screening at the monoclonal level of phage could be carried out.

### 2. Screening and sequencing of phage single clones

The experimental procedure was as follows:
From the first round of phage library enriched from coated HSA, 96 monoclonal colonies were selected for phage expression, and the HSA protein was coated for ELISA binding activity assay.

From the second round of phage library enriched from coated MSA, 96 monoclonal colonies were selected for phage expression, and HSA, MSA, and monkey albumin were separately coated for ELISA binding activity assay.

The antigens HSA, MSA, and monkey albumin were coated at a protein concentration of 0.5 µg/mL at 4 °C overnight. Blocking: 3% milk powder, 200 µL/well, and room temperature for 1 h.

The 10-fold diluted phage expression supernatant was added at 100 µL/well and incubated at room temperature for 1 h. The detection antibody Anti-M13 Antibody (HRP-labeled) (concentration: 0.2 µg/mL) was added at 100 µL/well and incubated at room temperature for 1 h.

A TMB chromogenic solution was added at 200 µL/well for color development for 20 min, and a stopping solution was added at 50 µL/well. The OD450 was measured using a microplate reader.

From the 96 phage clones from the first round of phage library enriched from coated HSA, one clone was selected and numbered 2049. From the 96 phage clones from the second round of phage library enriched from coated MSA, three clones were selected and numbered 3004, 3005, and 3007, respectively. The ELISA results are shown in Table 5 and Table 6.

**Table 5. Detection of activity of phage single clone 2049 binding to HSA**

| Phage clone number | OD450 | |
|---|---|---|
| | Coated with HSA at 0.5 µg/mL | Coated with 3% milk powder for control |
| 2049 | 2.3808 | 0.0671 |

**Table 6. Detection of activity of phage single clones 3004, 3005, and 3007 binding to HSA, MSA, and monkey albumin**

| Phage clone number | OD450 | | | |
|---|---|---|---|---|
| | Coated with MSA at 0.5 µg/mL | Coated with HSA at 0.5 µg/mL | Coated with monkey albumin at 0.5 µg/mL | Coated with 3% milk powder for control |
| 3004 | 2.5845 | 2.4568 | 2.3331 | 0.0505 |
| 3005 | 2.9804 | 4.3634 | 2.7835 | 0.0556 |
| 3007 | 2.4649 | 2.8373 | 1.5561 | 0.0374 |

The sequences of four phage clones were obtained by DNA sequencing. The amino acid sequences of CDR-H1, CDR-H2, and CDR-H3 of four antibodies are shown in Table 7, and the amino acid and nucleotide sequences of four nanobodies are shown in Table 8.

**Table 7. Amino acid sequences of CDRs of four anti-albumin nanobodies**

| Candidate antibody number | SEQ ID NO: x | CDR-H1 | SEQ ID NO: x | CDR-H2 | SEQ ID NO: x | CDR-H3 |
|---|---|---|---|---|---|---|
| 2049 | 1 | YYAIG | 3 | CINSSGYSTNYANSVKG | 7 | AALECRTYEYDY |
| 3004 | 1 | YYAIG | 4 | CISSSGGSTNYADSVKG | 8 | ALLECRRDYRYLEG |
| 3005 | 2 | NYYMS | 5 | GISVDGSFLDYADAVKG | 9 | ASGPQGLRLGAP |
| 3007 | 2 | NYYMS | 6 | GISIDSSILDYTDAVKG | 9 | ASGPQGLRLGAP |

**Table 8. Amino acid sequences and nucleotide sequences of four anti-albumin nanobodies**

| SEQ ID NO: x | Antibody number | Antibody amino acid sequence |
|---|---|---|
| 10 | 2049 | HVQLVESGGGLVQPGGSLRLSCAASGFTLTYYAIGWFRQAPGKEREGVLCINSSGYS TNYANSVKGRFTISRDNAKNTVYLQMNSLKPEDTAVYYCAAAALECRTYEYDYWG QGTQVTVSS |
| 11 | 3004 | QLQLVESGGGLVQPGGSLRLSCAASGLTLDYYAIGWFRQAPGKEREGVSCISSSGGS TNYADSVKGRFTISRDNAKNTVYLQMNSLKPEDTAVYYCAAALLECRRDYRYLEG WGQGTLVTVSS |
| 12 | 3005 | QVQLVESGGGLAQPGGSLRLSCAASGFTFSNYYMSWARQAPGKGLEWVSGISVDG SFLDYADAVKGRFTISRDNAKNTVYLQMNSLKPEDTALYYCARASGPQGLRLGAPR GQGTQVTVSS |
| 13 | 3007 | QVQLVESGGGLAQPGGSLRLSCAASGFTFSNYYMSWARQAPGKGLEWVSGISIDSSI LDYTDAVKGRFTISRDNAKNTVYLQMNSLKPEDTALYYCARASGPQGLRLGAPRG QGTQVTVSS |

| SEQ ID NO: x | Antibody number | Antibody nucleotide sequence |
|---|---|---|
| 63 | 2049 | CATGTGCAGCTGGTGGAGTCCGGGGGAGGCTTGGTGCAGCCTGGGGGGTCTCTG AGACTCTCCTGTGCAGCCTCTGGATTCACTTTGACTTATTATGCCATAGGCTGGTT CCGCCAGGCCCCAGGGAAGGAGCGTGAGGGGGTCTTATGTATTAATAGTAGTGGT TATAGCACAAACTATGCAAACTCCGTGAAGGGCCGATTCACCATCTCCAGAGACA ACGCCAAGAACACGGTGTATCTGCAAATGAACAGCCTGAAACCTGAAGACACGG CCGTTTATTACTGTGCAGCAGCCGCCCTTGAATGTCGGACCTATGAGTATGACTAC TGGGGCCAGGGGACCCAGGTCACCGTCTCCTCA |
| 64 | 3004 | CAGTTGCAGCTCGTGGAGTCCGGTGGAGGCTTGGTGCAGCCTGGGGGGTCTCTG AGACTCTCCTGTGCAGCCTCTGGACTCACTTTGGATTATTATGCTATAGGCTGGTT CCGCCAGGCCCCAGGGAAGGAGCGTGAGGGGGTCTCATGTATTAGTAGTAGTGG TGGTAGCACAAACTATGCAGACTCCGTGAAGGGCCGATTCACCATCTCCAGAGA CAACGCCAAGAACACGGTGTATCTGCAAATGAACAGCCTGAAACCTGAGGACAC GGCCGTTTATTACTGTGCAGCAGCCCTTTTAGAATGTCGTCGAGACTACAGGTATC TCGAAGGTTGGGGCCAGGGCACCCTGGTCACTGTCTCCTCA |
| 65 | 3005 | CAGGTGCAGCTGGTGGAGTCTGGAGGAGGCTTGGCGCAGCCTGGGGGGTCTCTG AGACTCTCCTGTGCAGCCTCTGGATTCACCTTCAGTAACTATTACATGAGTTGGGC TCGCCAGGCTCCAGGAAAGGGACTCGAGTGGGTGTCCGGTATTAGTGTTGATGG TAGTTTCCTTGACTATGCAGACGCCGTGAAGGGCCGATTCACCATCTCCAGAGAC AACGCCAAGAACACGGTGTATCTGCAAATGAACAGCCTGAAACCTGAGGACACG GCACTGTATTACTGTGCAAGAGCCTCTGGGCCCCAGGGACTACGCCTTGGGGCCC CCCGGGGCCAGGGGACCCAGGTCACCGTCTCCTCA |
| 66 | 3007 | CAGGTGCAGCTGGTGGAGTCTGGAGGAGGCTTGGCGCAGCCTGGGGGGTCTCTG AGACTCTCCTGTGCAGCCTCTGGATTCACCTTCAGTAACTATTACATGAGTTGGGC CCGCCAGGCTCCAGGAAAGGGACTCGAGTGGGTGTCCGGTATTAGTATTGATAGT AGTATCCTAGACTATACAGACGCCGTGAAGGGCCGATTCACCATCTCCAGAGACA ACGCCAAGAACACGGTGTATCTGCAAATGAACAGCCTGAAACCTGAGGACACGG |
| | | CACTGTATTACTGTGCAAGAGCCTCTGGGCCCCAGGGACTACGCCTTGGGGCCCC CCGGGGCCAGGGGACCCAGGTCACCGTCTCCTCA |

### Example 3: Detection of Activity of Anti-Albumin Candidate Antibody Binding to Albumin of Different Species

### 1. Expression of anti-albumin candidate antibody

The four candidate antibodies were expressed in mammalian cells. After protein purification, an antigen binding activity assay was performed to validate the activity of the candidate antibodies in binding to albumin at the protein level.

Through gene synthesis, the expression plasmids of His-tagged antibodies 2049, 3004, 3005, and 3007 and the positive control antibody Alb Nb were separately constructed. The positive control Alb Nb antibody was a nanobody against human serum albumin. The amino acid sequence of the antibody was from SEQ ID NO: 62 in the patent US 2007/0269422 A1, and the amino acid sequence was:


 (SEQ ID NO: 62).

The tPA secretion signal peptide MDAMKRGLCCVLLLCGAVFVSPS (SEQ ID NO: 37) was added to the N terminus of the above antibody, and the linker GGGGS (SEQ ID NO: 41) and 6xHis were added to the C terminus of the antibody. The gene sequence was linked to the expression vector pCDNA3.1(+) through the enzyme cutting sites NheI and XbaI. After successful sequencing of the plasmids, large-scale endotoxin-free extraction was performed, and the transfection reagent polyethylenimine (PEI) was used for transient expression of suspended 293F cells and Ni column affinity purification.

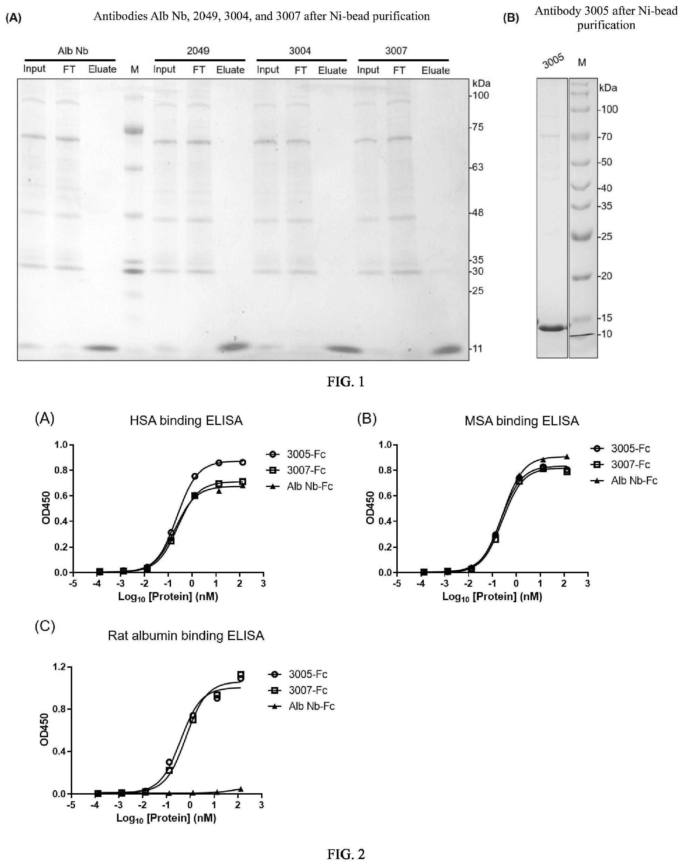

See FIG. 1 (A) for SDS-PAGE staining of cell culture medium supernatant (Input) transiently expressed by His-tagged antibodies Alb Nb, 2049, 3004, and 3007, flow-through (FT) that could not bind to the Ni column, and eluted purified product (Eluate). See FIG. 1 (B) for SDS-PAGE staining results of the product of His-tagged antibody 3005 purified by the Ni column. Their molecular weights were in line with the expected value (about 15 kDa).

Also, fusion proteins of antibodies 3005 and 3007 and the positive control antibody Alb Nb with Fc were constructed. The tPA secretion signal peptide MDAMKRGLCCVLLLCGAVFVSPS (SEQ ID NO: 37) was added to the N terminus of the antibody coding sequence by primer design. The gene fragment was inserted into the mammalian cell expression vector pCDNA3.1(+) by adding the amino acid sequence of human IgG1 Fc to the C terminus of the antibody sequence by homologous recombination:

PPCPAPELLGGPSVFLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVSLTCLVKGFYPSDIAV
EWESNGQPENNYKTTPPVLDSDGSFFLYSKLTVDKSRWQQGNVFSCSVMHEALHNHYTQKSLSLSPGK (SEQ ID
NO: 38).

The amino acid and nucleotide sequences of the anti-albumin nanobody-Fc fusion protein are shown in Table 9.

**Table 9. Amino acid sequence and nucleotide sequence of anti-albumin nanobody-Fc fusion protein**

| SEQ ID NO: x | Name of antibody | Antibody amino acid sequence |
|---|---|---|
| 14 | 3005-Fc | QVQLVESGGGLAQPGGSLRLSCAASGFTFSNYYMSWARQAPGKGLEWVSGISVDGSFL DYADAVKGRFTISRDNAKNTVYLQMNSLKPEDTALYYCARASGPQGLRLGAPRGQGTQ VTVSSPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAK GQPREPQVYTLPPSRDELTKNQVSLTCLVKGFYPSDIAVEWESNGQPENNYKTTPPVLDS DGSFFLYSKLTVDKSRWQQGNVFSCSVMHEALHNHYTQKSLSLSPGK |
| 15 | 3007-Fc | QVQLVESGGGLAQPGGSLRLSCAASGFTFSNYYMSWARQAPGKGLEWVSGISIDSSILDY |
| | | TDAVKGRFTISRDNAKNTVYLQMNSLKPEDTALYYCARASGPQGLRLGAPRGQGTQVT VSSPPCPAPELLGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPGK |

| SEQ ID NO: x | Name of antibody | Antibody nucleotide sequence |
|---|---|---|
| 67 | 3005-Fc | CAGGTGCAGCTGGTGGAGTCTGGAGGAGGCTTGGCGCAGCCTGGGGGGTCTCTGAG ACTCTCCTGTGCAGCCTCTGGATTCACCTTCAGTAACTATTACATGAGTTGGGCTCGCC AGGCTCCAGGAAAGGGACTCGAGTGGGTGTCCGGTATTAGTGTTGATGGTAGTTTCCT TGACTATGCAGACGCCGTGAAGGGCCGATTCACCATCTCCAGAGACAACGCCAAGAA CACGGTGTATCTGCAAATGAACAGCCTGAAACCTGAGGACACGGCACTGTATTACTGT GCAAGAGCCTCTGGGCCCCAGGGACTACGCCTTGGGGCCCCCCGGGGCCAGGGGAC CCAGGTCACCGTCTCCTCACCTCCTTGTCCTGCCCCCGAGCTGCTGGGCGGACCTTCT GTGTTCCTGTTCCCTCCCAAGCCCAAGGACACCCTGATGATCAGCAGGACCCCCGAG GTGACCTGCGTGGTGGTGGACGTGTCCCACGAGGACCCTGAGGTGAAGTTCAATTGG TACGTGGATGGCGTGGAGGTGCACAATGCCAAGACAAAGCCCAGAGAGGAGCAGTA CAATTCCACCTACAGAGTGGTGAGCGTGCTGACAGTGCTGCACCAGGACTGGCTGAA TGGCAAGGAGTACAAGTGTAAGGTGTCCAACAAGGCCCTGCCTGCCCCCATCGAGAA GACCATCAGCAAGGCCAAGGGCCAGCCTAGGGAGCCCCAGGTGTACACCCTGCCTCC TTCCAGGGACGAGCTGACAAAGAACCAGGTGAGCCTGACCTGCCTGGTGAAGGGCT TCTACCCCAGCGACATCGCCGTGGAGTGGGAGTCCAATGGCCAGCCTGAGAACAACT ACAAGACAACACCCCCTGTGCTGGATAGCGACGGCTCCTTCTTCCTGTACTCCAAGCT GACAGTGGACAAGTCCAGGTGGCAGCAGGGCAACGTGTTCAGCTGCTCCGTGATGC ACGAGGCCCTGCACAATCACTACACACAGAAGAGCCTGAGCCTGAGCCCCGGCAAG |
| 68 | 3007-Fc | CAGGTGCAGCTGGTGGAGTCTGGAGGAGGCTTGGCGCAGCCTGGGGGGTCTCTGAG ACTCTCCTGTGCAGCCTCTGGATTCACCTTCAGTAACTATTACATGAGTTGGGCTCGCC AGGCTCCAGGAAAGGGACTCGAGTGGGTGTCCGGTATTAGTGTTGATGGTAGTTTCCT TGACTATGCAGACGCCGTGAAGGGCCGATTCACCATCTCCAGAGACAACGCCAAGAA CACGGTGTATCTGCAAATGAACAGCCTGAAACCTGAGGACACGGCACTGTATTACTGT GCAAGAGCCTCTGGGCCCCAGGGACTACGCCTTGGGGCCCCCGGGGCCAGGGGAC CCAGGTCACCGTCTCCTCACCTCCTTGTCCTGCCCCCGAGCTGCTGGGCGGACCTTCT GTGTTCCTGTTCCCTCCCAAGCCCAAGGACACCCTGATGATCAGCAGGACCCCCGAG GTGACCTGCGTGGTGGTGGACGTGTCCCACGAGGACCCTGAGGTGAAGTTCAATTGG TACGTGGATGGCGTGGAGGTGCACAATGCCAAGACAAAGCCCAGAGAGGAGCAGTA CAATTCCACCTACAGAGTGGTGAGCGTGCTGACAGTGCTGCACCAGGACTGGCTGAA TGGCAAGGAGTACAAGTGTAAGGTGTCCAACAAGGCCCTGCCTGCCCCCATCGAGAA GACCATCAGCAAGGCCAAGGGCCAGCCTAGGGAGCCCCAGGTGTACACCCTGCCTCC TTCCAGGGACGAGCTGACAAAGAACCAGGTGAGCCTGACCTGCCTGGTGAAGGGCT TCTACCCCAGCGACATCGCCGTGGAGTGGGAGTCCAATGGCCAGCCTGAGAACAACT ACAAGACAACACCCCCTGTGCTGGATAGCGACGGCTCCTTCTTCCTGTACTCCAAGCT GACAGTGGACAAGTCCAGGTGGCAGCAGGGCAACGTGTTCAGCTGCTCCGTGATGC ACGAGGCCCTGCACAATCACTACACACAGAAGAGCCTGAGCCTGAGCCCCGGCAAG CAGGTGCAGCTGGTGGAGTCTGGAGGAGGCTTGGCGCAGCCTGGGGGGTCTCTGAG ACTCTCCTGTGCAGCCTCTGGATTCACCTTCAGTAACTATTACATGAGTTGGGCCCGCC AGGCTCCAGGAAAGGGACTCGAGTGGGTGTCCGGTATTAGTATTGATAGTAGTATCCT AGACTATACAGACGCCGTGAAGGGCCGATTCACCATCTCCAGAGACAACGCCAAGAA CACGGTGTATCTGCAAATGAACAGCCTGAAACCTGAGGACACGGCACTGTATTACTGT GCAAGAGCCTCTGGGCCCCAGGGACTACGCCTTGGGGCCCCCGGGGCCAGGGGAC CCAGGTCACCGTCTCCTCACCTCCTTGTCCTGCCCCCGAGCTGCTGGGCGGACCTTCT GTGTTCCTGTTCCCTCCCAAGCCCAAGGACACCCTGATGATCAGCAGGACCCCCGAG GTGACCTGCGTGGTGGTGGACGTGTCCCACGAGGACCCTGAGGTGAAGTTCAATTGG TACGTGGATGGCGTGGAGGTGCACAATGCCAAGACAAAGCCCAGAGAGGAGCAGTA CAATTCCACCTACAGAGTGGTGAGCGTGCTGACAGTGCTGCACCAGGACTGGCTGAA TGGCAAGGAGTACAAGTGTAAGGTGTCCAACAAGGCCCTGCCTGCCCCCATCGAGAA GACCATCAGCAAGGCCAAGGGCCAGCCTAGGGAGCCCCAGGTGTACACCCTGCCTCC TTCCAGGGACGAGCTGACAAAGAACCAGGTGAGCCTGACCTGCCTGGTGAAGGGCT TCTACCCCAGCGACATCGCCGTGGAGTGGGAGTCCAATGGCCAGCCTGAGAACAACT ACAAGACAACACCCCCTGTGCTGGATAGCGACGGCTCCTTCTTCCTGTACTCCAAGCT GACAGTGGACAAGTCCAGGTGGCAGCAGGGCAACGTGTTCAGCTGCTCCGTGATGC ACGAGGCCCTGCACAATCACTACACACAGAAGAGCCTGAGCCTGAGCCCCGGCAAG |

After successful sequencing of the expression plasmid of the Fc fusion protein, large-scale endotoxin-free extraction was performed, and the target protein was obtained by transient expression of suspended 293F cells and Protein A affinity purification.

### 2. Detection of activity of anti-albumin candidate antibody binding to albumin

Bio-layer interferometry (BLI) was used to assay the affinity of the monovalent nanobody binding to albumin from different species. Eight NTA sensors were used to bind to monovalent nanobodies (with a concentration of 1 µg/mL) with the same His tag. The antigens were separately human serum albumin HSA (Baxter AG), mouse albumin MSA (purchased from Equitech-bio, Cat. No.: MSA62-1000), and rat albumin (purchased from abcam, Cat. No.: ab198656). In the BLI experiment, 8 concentration gradients of the antigens were set to 200, 100, 50, 25, 12.5, 6.25, and 3.125 nM. The antigen and the antibody were bound for 180 s, and dissociated for 360 s.

Table 10 shows the binding rate (ka), dissociation rate (kd), and equilibrium dissociation constant (K_{D}) of his-tagged monovalent nanobodies 2049, 3004, 3005, and 3007 binding to albumin of different species after "1:1" model fitting.

**Table 10. Binding rate (ka), dissociation rate (kd), and equilibrium dissociation constant (K_{D}) of nanobodies 2049, 3004, 3005, and 3007 binding to albumin of different species**

| Antibody | Antigen | KD (M) | ka (1/Ms) | kd (1/s) | Full R^2 |
|---|---|---|---|---|---|
| 2049 | HSA | 5.79E-09 | 2.02E+05 | 1.17E-03 | 0.9967 |
| | MSA | 2.34E-09 | 1.36E+05 | 3.19E-04 | 0.9962 |
| | Rat albumin | No data | No data | No data | No data |
| 3004 | HSA | 8.04E-09 | 3.01E+05 | 2.42E-03 | 0.9812 |
| | MSA | 3.02E-10 | 1.84E+05 | 5.57E-05 | 0.996 |
| | Rat albumin | No data | No data | No data | No data |
| 3005 | HSA | 4.05E-09 | 1.87E+05 | 7.57E-04 | 0.9983 |
| | MSA | 5.78E-09 | 2.71E+05 | 1.57E-03 | 0.9858 |
| | Rat albumin | 5.32E-09 | 1.55E+05 | 8.26E-04 | 0.9982 |
| 3007 | HSA | 3.85E-09 | 1.79E+05 | 6.90E-04 | 0.9986 |
| | MSA | 1.15E-08 | 1.27E+05 | 1.46E-03 | 0.9969 |
| | Rat albumin | 4.08E-09 | 1.03E+05 | 4.18E-04 | 0.9985 |

The data showed that 2049, 3004, 3005, and 3007 all bound to HSA and MSA with high affinity, and 3005 and 3007 also bound to rat albumin.

The binding activity of 3005 and 3007 to albumin from different species were compared again by ELISA method. The experimental method was as follows:
The antigen was diluted with ELISA coating buffer. The final concentration of human serum albumin HSA (Baxter AG) was 1 µg/mL. The final concentration of mouse albumin MSA (purchased from Equitech-bio, Cat. No.: MSA62-1000) was 2 µg/mL.

The final concentration of rat albumin (purchased from abcam, Cat. No.: ab198656) was 2 µg/mL. The antigen dilution was added to a microplate at 100 µL/well and coated overnight at 4 °C. After the plate was blocked with 5% skimmed milk powder, the gradient dilutions (0.0001, 0.001, 0.01, 0.1, 1, 10, and 100 nM) of the candidate antibodies 3005-Fc and 3007-Fc, and the positive control antibody Alb Nb-Fc prepared with PBST (PBS containing 0.1% Tween 20, pH=7.4) were added. The plate was incubated at 37 °C for 1 h and then washed. Subsequently, HRP-labeled goat anti-human IgG Fc antibody (purchased from Abbkine, Cat. No.: A21050) was added, and the plate was incubated at room temperature for 45 min. After the plate was washed, 100 µL of TMB (purchased from Tiangen Biotech, Cat. No.: PA 107-01) was added to each well for color development at 37 °C for 15 min, followed by the addition of 50 µL of stopping solution. The absorbance at 450 nm (OD450) was measured using a microplate reader.

The ELISA results for the binding of 3005-Fc, 3007-Fc, and Alb Nb-Fc to albumin of different species are shown in FIG. 2. The EC50 of the binding ELISA experiment of 3005-Fc and 3007-Fc is summarized in Table 11.

**Table 11. Summary of ELISA experiment EC50 of 3005-Fc and 3007-Fc binding to albumin of different species**

| Protein | EC50 (nM) for binding to HSA | EC50 (nM) for binding to MSA | EC50 (nM) for binding to rat albumin |
|---|---|---|---|
| 3005-Fc | 0.24 | 0.23 | 0.40 |
| 3007-Fc | 0.25 | 0.26 | 0.68 |

Referring to Table 11, the results show that both antibodies 3005-Fc and 3007-Fc could effectively bind to HSA and MSA. Referring to FIG. 2, the activity of the positive control antibody Alb Nb-Fc binding to rat albumin was very weak, but both antibodies 3005-Fc and 3007-Fc had good binding activity to rat serum albumin, and the binding activity of antibody 3005-Fc to rat albumin was better than that of 3007-Fc. Therefore, 3005 antibody was preferably selected for subsequent studies.

### Example 4: Humanization Engineering of Anti-Albumin Candidate Antibody

### 1. Humanization engineering, expression, and purification of candidate antibody

Humanization engineering was performed by grafting the CDRs of the antibody. Humanization engineering of the antibody was performed using the candidate antibody 3005 (SEQ ID NO: 12) as the starting antibody. The sequence of the candidate antibody 3005 was first compared with human antibody sequences for homology using the antibody databases IGBLAST and IMGT, and subsequently, the human antibody framework region sequence with high homology were combined with the 3 CDR sequences (SEQ ID NO: 2, SEQ ID NO: 5, and SEQ ID NO: 9) of the candidate antibody 3005 to construct humanized antibodies.

The secretion signal peptide was added to the N terminus of the antibody, and the linker GGGGS (SEQ ID NO: 41) and 6xHis were added to the C terminus of the antibody. The coding DNA sequence was obtained by gene synthesis, and linked to the expression vector pCDNA3.1(+) through the enzyme cutting sites NheI and Xbal. The plasmids were sequenced successfully, and large-scale endotoxin-free extraction was performed. Transient expression and Ni column affinity purification were performed in suspended 293F cells.

The amino acid and nucleotide sequences of the humanization engineered protein of antibody 3005 are shown in Table 12.

**Table 12. Amino acid sequences and nucleotide sequences of 3005 humanized antibody**

| SEQ ID NO: x | Name of antibody | Antibody amino acid sequence |
|---|---|---|
| 16 | 3005Hz3 | EVQLVESGGGLVQPGGSLRLSCAASGFTFSNYYMSWVRQAPGKGLEWVSGIS VDGSFLDYADAVKGRFTISRDNAKNTLYLQMNSLRPEDTAVYYCARASGPQG LRLGAPRGQGTLVTVSS |
| 17 | 3005Hz4 | QVQLVESGGGLVKPGGSLRLSCAASGFTFSNYYMSWIRQAPGKGLEWVSGIS VDGSFLDYADAVKGRFTISRDNAKNSLYLQMNSLRAEDTAVYYCARASGPQG LRLGAPRGQGTQVTVSS |
| 18 | 3005Hz5 | EVQLVESGGGLVQPGGSLRLSCAASGFTFSNYYMSWIRQAPGKGLEWVSGIS VDGSFLDYADAVKGRFTISRDNAKNSLYLQMNSLRPEDTAVYYCARASGPQG LRLGAPRGQGTLVTVSS |
| 19 | 3005Hz6 | EVQLVESGGGLVQPGGSLRLSCAASGFTFSNYYMSWARQAPGKGLEWVSGIS VDGSFLDYADAVKGRFTISRDNAKNSLYLQMNSLRPEDTAVYYCARASGPQG LRLGAPRGQGTLVTVSS |

| SEQ ID NO: x | Name of antibody | Antibody nucleotide sequence |
|---|---|---|
| 69 | 3005Hz3 | GAGGTGCAGCTGGTGGAAAGCGGCGGCGGACTGGTTCAACCTGGAGGAT CTCTGCGGCTGAGCTGTGCTGCTTCTGGCTTTACCTTCAGCAACTACTATAT GAGCTGGGTCAGACAGGCCCCTGGCAAAGGCCTGGAATGGGTGTCCGGCA TCAGCGTGGACGGCAGCTTCCTGGACTACGCCGATGCCGTGAAGGGCAGA TTCACAATCAGCAGAGATAATGCCAAGAACACCCTGTACCTGCAGATGAAC AGCCTGCGCCCCGAGGACACCGCCGTGTACTACTGCGCCAGAGCCTCTGG ACCTCAGGGCCTCCGGCTGGGCGCCCCAAGAGGCCAGGGCACACTGGTG ACCGTGTCCAGC CAAGTGCAGCTGGTTGAGAGCGGCGGAGGCCTGGTGAAACCTGGAGGAT CTCTGAGACTGTCTTGTGCTGCTTCTGGCTTTACATTCAGCAACTACTATAT GAGCTGGATCAGACAGGCCCCAGGCAAGGGCCTGGAATGGGTCAGCGGC ATCAGCGTGGACGGCAGCTTCCTGGACTACGCCGATGCCGTGAAGGGCAG |
| 70 | 3005Hz4 | CAAGTGCAGCTGGTTGAGAGCGGCGGAGGCCTGGTGAAACCTGGAGGAT CTCTGAGACTGTCTTGTGCTGCTTCTGGCTTTACATTCAGCAACTACTATAT GAGCTGGATCAGACAGGCCCCAGGCAAGGGCCTGGAATGGGTCAGCGGC ATCAGCGTGGACGGCAGCTTCCTGGACTACGCCGATGCCGTGAAGGGCAG |
| | | ATTCACAATCAGCAGAGATAATGCCAAGAACTCCCTGTACCTGCAGATGAA CAGCCTGCGGGCCGAGGACACCGCCGTGTACTACTGCGCCAGAGCCAGCG GACCTCAGGGCCTCCGGCTGGGCGCCCCCAGAGGCCAGGGCACCCAGGT GACCGTGTCCAGC |
| 71 | 3005Hz5 | GAGGTGCAGCTGGTGGAAAGCGGCGGAGGACTGGTCCAGCCCGGCGGAA GCCTGCGGCTCTCTTGTGCTGCTTCTGGCTTTACCTTCAGCAACTACTATAT GAGCTGGATCAGACAGGCCCCTGGCAAAGGCCTGGAATGGGTGTCCGGCA TCAGCGTGGACGGCAGCTTCCTGGACTACGCCGATGCCGTGAAGGGCAGA TTCACAATCAGCAGAGACAATGCCAAGAACAGCCTGTACCTGCAGATGAA CAGCCTGCGGCCTGAGGACACCGCCGTGTACTACTGCGCCAGAGCCAGCG GACCTCAAGGCCTGAGACTGGGCGCCCCAAGAGGCCAGGGCACCCTGGT GACAGTGTCCAGC |
| 72 | 3005Hz6 | GAAGTGCAGCTGGTCGAGAGCGGCGGAGGACTGGTGCAACCTGGCGGCA GCCTCAGACTGAGCTGTGCTGCTTCTGGCTTCACCTTCAGCAACTACTATAT GAGCTGGGCCAGACAGGCCCCAGGCAAAGGCCTGGAATGGGTTTCCGGC ATCAGCGTGGACGGCTCTTTCTGGACTACGCCGATGCCGTGAAGGGCAG ATTCACAATCTCTCGGGATAATGCCAAGAACAGCCTGTACCTGCAGATGAA CAGCCTGAGACCTGAGGACACCGCCGTGTACTACTGCGCCCGGGCCAGCG GCCCCCAGGGCCTGCGGCTGGGAGCCCCTAGAGGCCAGGGTACACTGGTG ACCGTGTCCAGC |

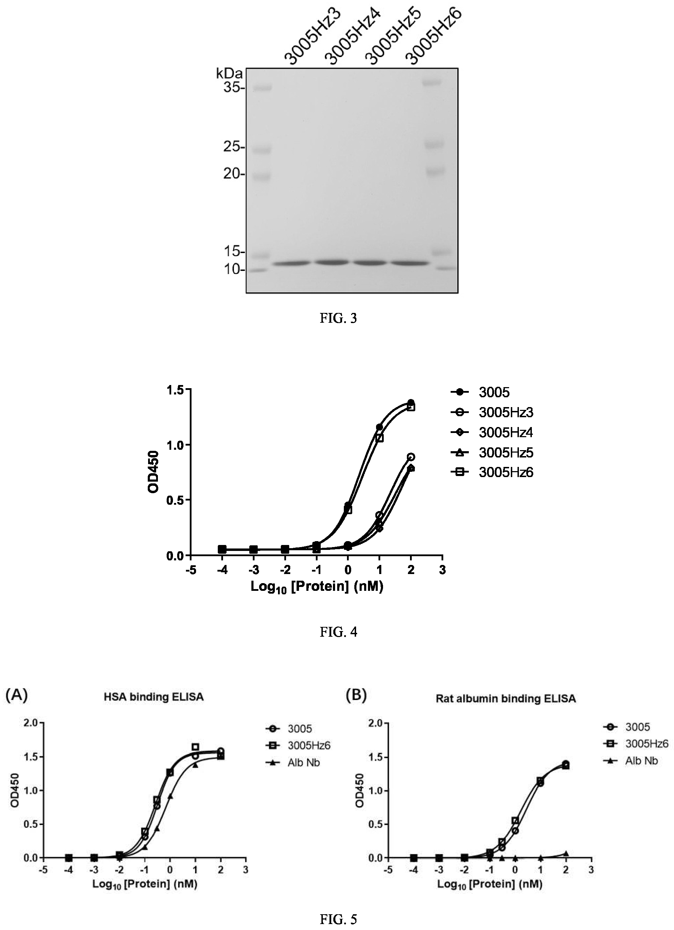

The His-tag-containing humanized protein was purified by Ni column and separated by SDS-PAGE electrophoresis, and the molecular weight was in line with the expected value (about 13 kDa). The Coomassie staining results are shown in FIG. 3.

### 2. Activity assay of humanized antibody

First, the activity of the original candidate antibody 3005 and the humanization engineered antibody binding to mouse albumin MSA was detected by ELISA method.

In the experiment, mouse albumin MSA (coated with a protein concentration of 5 µg/mL) was coated in a microplate at 100 µL/well at 4 °C overnight. After the plate was blocked with 5% skimmed milk powder, the dilutions (0.0001, 0.001, 0.01, 0.1, 1, 10, and 100 nM) containing His-tag proteins to be tested were prepared with PBST (PBS containing 0.1% Tween 20, pH=7.4). The proteins to be tested were the original antibody 3005 and the four humanization engineered antibodies. The protein dilution was added to the microplate. The plate was incubated at 37 °C for 1 h and washed. Subsequently, HRP-labeled mouse anti-His tag monoclonal antibody (purchased from Proteintech, Cat. No.: HRP-66005) was added, and the plate was incubated at room temperature for 45 min. After the plate was washed, 100 µL of TMB (purchased from Tiangen Biotech, Cat. No.: PA107-01) was added to each well for color development at 37 °C for 15 min, followed by the addition of 50 µL of stopping solution. The absorbance at 450 nm (OD450) was measured using a microplate reader.

The ELISA results for the binding of antibody 3005 and humanized antibodies thereof to MSA are shown in FIG. 4. The EC50 of the binding ELISA experiment is summarized in Table 13.

**Table 13. Summary of ELISA experiment EC50 and maximum binding value of antibody 3005 and humanized antibody thereof binding to MSA**

| Antibody | EC50 (nM) for binding to MSA | Maximum binding value OD450 |
|---|---|---|
| 3005 | 2.3 | 1.38 |
| 3005Hz3 | 22.0 | 0.89 |
| 3005Hz4 | 49.8 | 0.79 |
| 3005Hz5 | 31.6 | 0.80 |
| 3005Hz6 | 2.9 | 1.34 |

The results show that the activity of the humanized antibody 3005Hz6 binding to MSA was similar to that of the original antibody 3005.

The activity of the original candidate antibody 3005 and the humanized 3005Hz6 binding to HSA and rat albumin was detected by ELISA method.

In the experiment, HSA or rat albumin (coated with a protein concentration of 2 µg/mL) was coated in a microplate at 100 µL/well at 4 °C overnight. After the plate was blocked with 5% skimmed milk powder, the dilutions (0.0001, 0.001, 0.01, 0.1, 0.3, 1, 10, and 100 nM) containing His-tag proteins to be tested were prepared with PBST (PBS containing 0.1% Tween 20, pH=7.4). The proteins to be tested were the positive control antibody Alb Nb, the original antibody 3005, and the humanized antibody 3005Hz6. The protein dilution was added to the microplate. The plate was incubated at 37 °C for 1 h and washed. Subsequently, HRP-labeled mouse anti-His tag monoclonal antibody (purchased from Proteintech, Cat. No.: HRP-66005) was added, and the plate was incubated at room temperature for 45 min. After the plate was washed, 100 µL of TMB (purchased from Tiangen Biotech, Cat. No.: PA107-01) was added to each well for color development at 37 °C for 15 min, followed by the addition of 50 µL of stopping solution. The absorbance at 450 nm (OD450) was measured using a microplate reader.

The ELISA results are separately shown in (A) and (B) of FIG. 5. The EC50 values of the binding ELISA curve fit of FIG. 5 are shown in Table 14.

**Table 14. Summary of EC50 of candidate antibody 3005 and its humanized antibody 3005Hz6 binding to HSA and rat albumin**

| Name of antibody | EC50 (nM) for binding to HSA | EC50 (nM) for binding to rat albumin |
|---|---|---|
| 3005 | 0.31 | 2.74 |
| 3005Hz6 | 0.26 | 1.64 |
| Alb Nb | 0.66 | Weak binding |

As shown in FIG. 5 and Table 14, the binding activity of the humanized antibody 3005Hz6 to HSA and rat albumin was comparable to that of the original antibody 3005, and was stronger than that of the positive control antibody Alb Nb.

The binding rate (ka), dissociation rate (kd), and equilibrium dissociation constant (K_{D}) of the humanized antibody 3005Hz6 binding to HSA and MSA were detected by surface plasmon resonance (SPR) method.

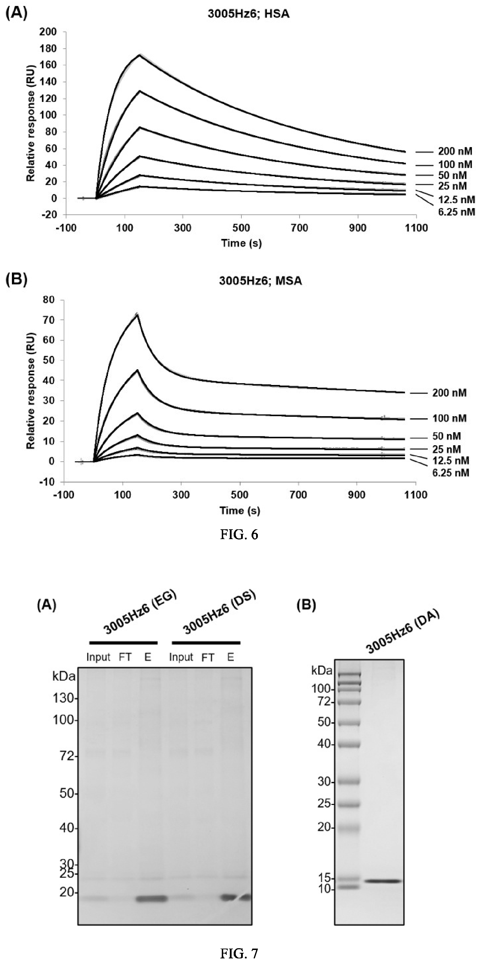

In the experiment, the Biacore 8K instrument (Cytiva) was used. His-tagged humanized antibody 3005Hz6 was captured using Series S Sensor Chip NTA. The analyte was HSA or MSA at gradient concentrations. The analyte was diluted with mobile phase buffer (composition: 10 mM HEPES, 150 mM NaCl, 0.05% v/v Tween-20, pH=7.4). The binding time was 150 s, and the dissociation time was 900 s. The binding and dissociation curves of the antibody 3005Hz6 to HSA are shown in FIG. 6 (A), and the binding and dissociation curves of the antibody 3005Hz6 to MSA are shown in FIG. 6 (B).

The "two state reaction" model was used to fit the binding rate (ka), dissociation rate (kd), and equilibrium dissociation constant (K_{D}) of the binding of 3005Hz6 to HSA and MSA. The results are shown in Table 15.

**Table 15. Binding rate (ka), dissociation rate (kd), and equilibrium dissociation constant (K_{D}) of humanized antibody 3005Hz6 with HSA and MSA**

| Ligand | Analyte Solution | ka1 (1/Ms) | kd1 (1/s) | ka2 (1/s) | kd2 (1/s) | KD (M) |
|---|---|---|---|---|---|---|
| 3005Hz6 | HSA | 9.04E+04 | 1.56E-03 | 3.67E-04 | 8.08E-04 | 1.19E-08 |
| 3005Hz6 | MSA | 7.57E+04 | 1.51E-02 | 5.01E-03 | 3.02E-04 | 1.14E-08 |

It can be seen from Table 15 that the affinity of the humanized antibody 3005Hz6 binding to HSA and MSA was similar, and the KD determined by SPR method was about 11 nM.

In combination with Table 10 of Example 3, the KD value of the original antibody 3005 to HSA and MSA determined by BLI method was about 5 nM, which was within 3-fold of the KD in Table 15. Therefore, it was considered that the affinity of the humanized antibody 3005Hz6 binding to HSA and MSA was similar to that of the original antibody 3005.

### Example 5: Engineering of Potential Chemical Modification Sites of Humanized Antibody 3005Hz6 and Activity Assay

### 1. Engineering scheme of potential chemical modification sites, expression, and purification of humanized antibody 3005Hz6

For the 3005Hz6 antibody, its CDR-H2 region (SEQ ID NO: 5) contains the amino acid motif DG. DG is more likely to promote the isomerization of aspartate D to isoaspartate than other amino acid motifs (such as DS and DA) and may alter antibody structure and antigen-binding activity. Aspartate isomerization may also accelerate proteolysis and enhance the immunogenicity of a protein.

Therefore, in this example, the amino acid combination DG in the CDR-H2 region of 3005Hz6 was modified by point mutation to reduce the risk of structural and activity changes in the protein. The point mutation scheme of the amino acid combination DG of 3005Hz6 was specifically as follows: Aspartate D was replaced with glutamic acid E, or glycine G was mutated into A with simple structure or S that is easy to form flexible structure. The numbers of the engineered antibodies and the amino acid sequences of their CDR regions are shown in Table 16. The full-length amino acid and nucleotide sequences of the mutants at the potential chemical modification sites of the humanized antibody 3005Hz6 are shown in Table 17.

**Table 16. Amino acid sequences of CDRs of mutants at potential chemical modification sites of humanized antibody 3005Hz6**

| Candidate antibody number | SEQ ID NO: x | CDR-H1 | SEQ ID NO: x | CDR-H2 | SEQ ID NO: x | CDR-H3 |
|---|---|---|---|---|---|---|
| 3005Hz6 (DA) | 2 | NYYMS | 20 | GISVDASFLDYADAVKG | 9 | ASGPQGLRLGAP |
| 3005Hz6 (DS) | 2 | NYYMS | 21 | GISVDSSFLDYADAVKG | 9 | ASGPQGLRLGAP |
| 3005Hz6 (EG) | 2 | NYYMS | 22 | GISVEGSFLDYADAVKG | 9 | ASGPQGLRLGAP |

**Table 17. Full-length amino acid sequences and nucleotide sequences of mutants at potential chemical modification sites of humanized antibody 3005Hz6**

| SEQ ID NO: x | Name of antibody | Full-length amino acid sequence of mutant of humanized antibody 3005Hz6 |
|---|---|---|
| 23 | 3005Hz6 (DA) | EVQLVESGGGLVQPGGSLRLSCAASGFTFSNYYMSWARQAPGKGLEWVSGISVD ASFLDYADAVKGRFTISRDNAKNSLYLQMNSLRPEDTAVYYCARASGPQGLRLG APRGQGTLVTVSS |
| 24 | 3005Hz6 (DS) | EVQLVESGGGLVQPGGSLRLSCAASGFTFSNYYMSWARQAPGKGLEWVSGISVD SSFLDYADAVKGRFTISRDNAKNSLYLQMNSLRPEDTAVYYCARASGPQGLRLGA PRGQGTLVTVSS |
| 25 | 3005Hz6 (EG) | EVQLVESGGGLVQPGGSLRLSCAASGFTFSNYYMSWARQAPGKGLEWVSGISVE GSFLDYADAVKGRFTISRDNAKNSLYLQMNSLRPEDTAVYYCARASGPQGLRLG APRGQGTLVTVSS |

| SEQ ID NO: x | Name of antibody | Antibody nucleotide sequence |
|---|---|---|
| 73 | 3005Hz6 (DA) | GAAGTGCAGCTGGTCGAGAGCGGTGGAGGACTGGTGCAACCTGGCGGAAGC CTCAGACTGAGCTGTGCTGCCTCTGGCTTCACCTTCAGCAACTACTATATGAGC TGGGCCAGACAGGCCCCAGGCAAAGGCCTGGAATGGGTTTCCGGCATCAGCG TGGACGCTAGCTTTCTGGACTACGCCGATGCCGTGAAGGGCAGATTCACAATC TCTCGGGATAATGCCAAGAACAGCCTGTACCTGCAGATGAACAGCCTGAGACC TGAGGACACCGCCGTGTACTACTGCGCAAGGGCTTCCGGTCCTCAGGGCCTGC |
| | | GGCTGGGAGCTCCCAGAGGTCAGGGTACACTGGTGACCGTGTCCAGC |
| 74 | 3005Hz6 (DS) | GAAGTGCAGCTGGTCGAGAGCGGTGGAGGACTGGTGCAACCTGGCGGAAGC CTCAGACTGAGCTGTGCTGCCTCTGGCTTCACCTTCAGCAACTACTATATGAGC TGGGCCAGACAGGCCCCAGGCAAAGGCCTGGAATGGGTTTCCGGCATCAGCG TGGACTCTAGCTTTCTGGACTACGCCGATGCCGTGAAGGGCAGATTCACAATC TCTCGGGATAATGCCAAGAACAGCCTGTACCTGCAGATGAACAGCCTGAGACC TGAGGACACCGCCGTGTACTACTGCGCAAGGGCTTCCGGTCCTCAGGGCCTGC GGCTGGGAGCTCCCAGAGGTCAGGGTACACTGGTGACCGTGTCCAGC |
| 75 | 3005Hz6 (EG) | GAAGTGCAGCTGGTCGAGAGCGGTGGAGGACTGGTGCAACCTGGCGGAAGC CTCAGACTGAGCTGTGCTGCCTCTGGCTTCACCTTCAGCAACTACTATATGAGC TGGGCCAGACAGGCCCCAGGCAAAGGCCTGGAATGGGTTTCCGGCATCAGCG TGGAGGGCAGCTTTCTGGACTACGCCGATGCCGTGAAGGGCAGATTCACAATC TCTCGGGATAATGCCAAGAACAGCCTGTACCTGCAGATGAACAGCCTGAGACC TGAGGACACCGCCGTGTACTACTGCGCAAGGGCTTCCGGTCCTCAGGGCCTGC GGCTGGGAGCTCCCAGAGGTCAGGGTACACTGGTGACCGTGTCCAGC |

The secretion signal peptide (SEQ ID NO: 37) was added to the N terminus of the mutant protein of antibody 3005Hz6, and the linker GGGGS (SEQ ID NO: 41) and 6xHis were added to the C terminus of the antibody. The coding DNA sequence was obtained by gene synthesis, and linked to the expression vector pCDNA3.1(+) through the enzyme cutting sites NheI and XhoI. The plasmids were sequenced successfully, and large-scale endotoxin-free extraction was performed. The suspended 293F cells were used for transient expression, and Ni column affinity purification was performed from the cell culture supernatant. During the purification process, for the cell culture supernatant (Input) of transiently expressed 3005Hz6 (EG) and 3005Hz6 (DS), the flow-through (FT) that could not bind to the Ni column, and the eluted purified product (E), their SDS-PAGE results are shown in FIG. 7 (A). The cell supernatant of transiently expressed 3005Hz6 (DA) protein was purified by Ni column and subsequently purified by cation exchange. The SDS-PAGE results of the purified protein are shown in FIG. 7 (B). The molecular weight of the three mutant proteins was 13 kDa.

### 2. Activity assay of engineered mutant at potential chemical modification sites of humanized antibody 3005Hz6

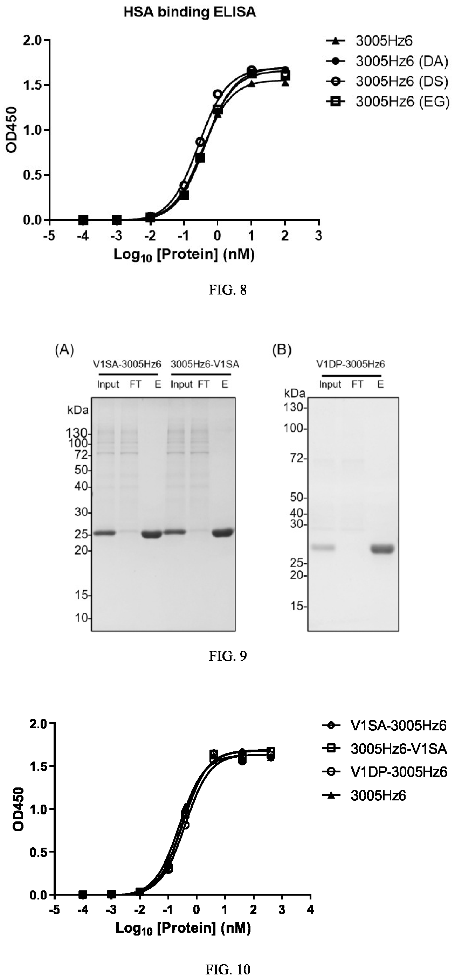

ELISA for HSA binding activity was performed on humanized antibody 3005Hz6 mutant proteins 3005Hz6 (DA), 3005Hz6 (DS), and 3005Hz6 (EG). In the ELISA experiment, 2 µg/mL HSA was coated, and 3005Hz6 or a mutant thereof at gradient concentrations was added. The level of HSA-binding antibodies was detected by HRP-labeled mouse anti-His tag monoclonal antibody (purchased from Proteintech, Cat. No.: HRP-66005). The ELISA experiment results are shown in FIG. 8, and the EC50 of the ELISA experiment is shown in Table 18.

**Table 18. Summary of EC50 of humanized antibody 3005Hz6 mutant proteins 3005Hz6 (DA), 3005Hz6 (DS), and 3005Hz6 (EG) binding to HSA**

| Name of antibody | EC50 (nM) for binding to HSA |
|---|---|
| 3005Hz6 | 0.35 |
| 3005Hz6 (DA) | 0.44 |
| 3005Hz6 (DS) | 0.28 |
| 3005Hz6 (EG) | 0.40 |

It can be seen from Table 18 that the humanized antibody 3005Hz6 and its mutant proteins had similar HSA binding activity.

### Example 6: Fusion Expression of Humanized Anti-Albumin Antibody with Other Antigen-Binding Proteins and Activity Assay

### I. Fusion expression and purification of humanized antibody 3005Hz6 and monovalent anti-VEGFA nanobody

After the humanized antibody 3005Hz6 was expressed in fusion with an antibody binding to other antigens, the fusion protein bound to albumin *in vivo* and the molecular weight increased. Alternatively, after binding to albumin, it participated in the binding of albumin to FcRn and the recycling mechanism, which could potentially prolong the half-life of the fusion protein *in vivo.*

Two anti-VEGFA nanobodies were selected: V1-SA1 (SEQ ID NO: 26) and V1-DP (SEQ ID NO: 27) (Table 19), which were expressed in fusion with the humanized antibody 3005Hz6, and the activity of the fusion proteins obtained in different tandem approaches was compared.

**Table 19. Anti-VEGFA nanobody sequence**

| SEQ ID NO: x | Name of anti-VEGFA nanobody | Amino acid sequence of anti-VEGFA nanobody |
|---|---|---|
| 26 | V1-SA1 | EVQLVESGGGLVQPGGSLRLSCAVSGRTFSSYTMGWFRQAPGKEREFVVA ISKGGYKYDSVSLEARFTISRDNAKNTVYLQMNSLRPEDTAVYYCAATRA YGSSRLRLADTYEYWGQGTLVTVSS |
| 27 | V1-DP | EVQLVESGGGLVQPGGSLRLSCAVSGRTFSSYTMGWFRQAPGKEREFVVA ISKGGYKYDSVSLEARFTISRDNSKNTVYLQMNSLRPEDTAVYYCAATRA YGSSRLRLADTYEYWGQGTLVTVSS |

When the humanized antibody 3005Hz6 was linked in series with monovalent anti-VEGFA nanobodies V1-SA1 (SEQ ID NO: 26) and V1-DP (SEQ ID NO: 27), linker GGS was used, and the amino acid sequence and nucleotide sequence of the fusion proteins are shown in Table 20.

**Table 20. Amino acid sequence and nucleotide sequence of fusion proteins of humanized antibody 3005Hz6 and monovalent anti-VEGFA nanobody**

| SEQ ID NO: x | Name of fusion protein | Amino acid sequence of fusion protein |
|---|---|---|
| 28 | V1SA-3005Hz6 | EVQLVESGGGLVQPGGSLRLSCAVSGRTFSSYTMGWFRQAPGKEREFVVAISKG GYKYDSVSLEARFTISRDNAKNTVYLQMNSLRPEDTAVYYCAATRAYGSSRLR LADTYEYWGQGTLVTVSSGGSEVQLVESGGGLVQPGGSLRLSCAASGFTFSNY YMSWARQAPGKGLEWVSGISVDGSFLDYADAVKGRFTISRDNAKNSLYLQMN SLRPEDTAVYYCARASGPQGLRLGAPRGQGTLVTVSS |
| 29 | 3005Hz6-V1SA | EVQLVESGGGLVQPGGSLRLSCAASGFTFSNYYMSWARQAPGKGLEWVSGISV DGSFLDYADAVKGRFTISRDNAKNSLYLQMNSLRPEDTAVYYCARASGPQGLR LGAPRGQGTLVTVSSGGSEVQLVESGGGLVQPGGSLRLSCAVSGRTFSSYTMG WFRQAPGKEREFVVAISKGGYKYDSVSLEARFTISRDNAKNTVYLQMNSLRPE DTAVYYCAATRAYGSSRLRLADTYEYWGQGTLVTVSS |
| 30 | V1DP-3005Hz6 | EVQLVESGGGLVQPGGSLRLSCAVSGRTFSSYTMGWFRQAPGKEREFVVAISKG GYKYDSVSLEARFTISRDNSKNTVYLQMNSLRPEDTAVYYCAATRAYGSSRLRL ADTYEYWGQGTLVTVSSGGSEVQLVESGGGLVQPGGSLRLSCAASGFTFSNYY MSWARQAPGKGLEWVSGISVDGSFLDYADAVKGRFTISRDNAKNSLYLQMNSL RPEDTAVYYCARASGPQGLRLGAPRGQGTLVTVSS |

| SEQ ID NO: x | Name of antibody | Antibody nucleotide sequence |
|---|---|---|
| 76 | V1SA-3005Hz6 | GAGGTGCAACTGGTGGAGTCCGGAGGTGGCCTGGTCCAGCCTGGCGGCAGC CTGAGACTCTCCTGCGCCGTGAGCGGCAGAACCTTCAGCAGCTACACCATG GGCTGGTTCAGACAAGCTCCAGGCAAAGAAAGGGAGTTCGTGGTGGCTATT AGCAAAGGCGGCTATAAGTACGATAGCGTGAGCCTGGAAGCTCGGTTCACC ATTAGCAGAGATAACGCCAAGAACACCGTGTACCTGCAAATGAATAGCCTGA GACCTGAGGATACCGCCGTGTACTACTGTGCCGCCACAAGAGCCTATGGCAG CTCCAGGCTCAGACTGGCTGATACCTACGAATATTGGGGCCAAGGCACCCTG GTGACAGTCAGCAGCGGCGGATCCGAAGTGCAGCTGGTCGAGAGCGGCGG AGGACTGGTGCAACCTGGCGGCAGCCTCAGACTGAGCTGTGCTGCTTCTGG CTTCACCTTCAGCAACTACTATATGAGCTGGGCCAGACAGGCCCCAGGCAAA |
| | | GGCCTGGAATGGGTTTCCGGCATCAGCGTGGACGGCTCTTTTCTGGACTACG CCGATGCCGTGAAGGGCAGATTCACAATCTCTCGGGATAATGCCAAGAACAG CCTGTACCTGCAGATGAACAGCCTGAGACCTGAGGACACCGCCGTGTACTAC TGCGCCCGGGCCAGCGGCCCCCAGGGCCTGCGGCTGGGAGCCCCTAGAGGC CAGGGTACACTGGTGACCGTGTCCAGC |
| 77 | 3005Hz6-V1SA | GAAGTGCAGCTGGTCGAGAGCGGCGGAGGACTGGTGCAACCTGGCGGCAG CCTCAGACTGAGCTGTGCTGCTTCTGGCTTCACCTTCAGCAACTACTATATGA GCTGGGCCAGACAGGCCCCAGGCAAAGGCCTGGAATGGGTTTCCGGCATCA GCGTGGACGGCTCTTTTCTGGACTACGCCGATGCCGTGAAGGGCAGATTCAC AATCTCTCGGGATAATGCCAAGAACAGCCTGTACCTGCAGATGAACAGCCTG AGACCTGAGGACACCGCCGTGTACTACTGCGCCCGGGCCAGCGGCCCCCAG GGCCTGCGGCTGGGAGCCCCTAGAGGCCAGGGTACACTGGTGACCGTGTCC AGCGGCGGATCCGAGGTGCAACTGGTGGAGTCCGGAGGTGGCCTGGTCCAG CCTGGCGGCAGCCTGAGACTCTCCTGCGCCGTGAGCGGCAGAACCTTCAGC AGCTACACCATGGGCTGGTTCAGACAAGCTCCAGGCAAAGAAAGGGAGTTC GTGGTGGCTATTAGCAAAGGCGGCTATAAGTACGATAGCGTGAGCCTGGAAG CTCGGTTCACCATTAGCAGAGATAACGCCAAGAACACCGTGTACCTGCAAAT GAATAGCCTGAGACCTGAGGATACCGCCGTGTACTACTGTGCCGCCACAAGA GCCTATGGCAGCTCCAGGCTCAGACTGGCTGATACCTACGAATATTGGGGCC AAGGCACCCTGGTGACAGTCAGCAGC |
| 78 | V1DP-3005Hz6 | GAAGTTCAGTTGGTCGAATCAGGAGGAGGATTGGTTCAACCTGGTGGTTCTC TCAGATTGTCTTGCGCTGTGAGTGGCAGGACATTCAGCAGCTACACTATGGG ATGGTTTCGCCAGGCTCCAGGCAAGGAGAGAGAGTTTGTAGTCGCCATTTCT AAGGGGGGATACAAGTACGACTCTGTGAGCCTGGAGGCTAGGTTCACAATC AGCAGGGATAACTCTAAGAATACCGTCTACCTGCAGATGAATAGCCTCAGAC CTGAGGACACTGCCGTCTACTACTGCGCTGCCACTCGGGCTTACGGTTCCAG CAGGCTTAGGCTGGCCGATACATATGAATACTGGGGCCAGGGCACCCTTGTT ACAGTGAGCAGTGGCGGATCCGAAGTGCAGCTGGTCGAGAGCGGCGGAGG ACTGGTGCAACCTGGCGGCAGCCTCAGACTGAGCTGTGCTGCTTCTGGCTTC ACCTTCAGCAACTACTATATGAGCTGGGCCAGACAGGCCCCAGGCAAAGGC CTGGAATGGGTTTCCGGCATCAGCGTGGACGGCTCTTTTCTGGACTACGCCG ATGCCGTGAAGGGCAGATTCACAATCTCTCGGGATAATGCCAAGAACAGCCT GTACCTGCAGATGAACAGCCTGAGACCTGAGGACACCGCCGTGTACTACTG CGCCCGGGCCAGCGGCCCCCAGGGCCTGCGGCTGGGAGCCCCTAGAGGCCA GGGTACACTGGTGACCGTGTCCAGC |

The secretion signal peptide was added to the N terminus of the fusion protein, and the linker GGGGS and 6xHis were added to the C terminus of the antibody. The coding DNA sequence was obtained by gene synthesis (see Table 20), and linked to the expression vector pCDNA3.1(+) through the enzyme cutting sites NheI and XhoI. The plasmids were sequenced successfully, and large-scale endotoxin-free extraction was performed. The suspended 293F cells were used for transient expression, and Ni column affinity purification was performed from the cell culture supernatant. During the purification process, for the cell culture supernatant after transient transfection of each protein (Input), the flow-through (FT) that could not bind to the Ni column, and the eluted purified product, their SDS-PAGE results are shown in FIG. 9, and the molecular weight of the target protein was about 28 kDa.

### II. Activity assay of fusion protein of humanized antibody 3005Hz6 and monovalent anti-VEGFA nanobody

### 1. Detection of whether the two nanobody elements constituting the fusion protein maintain their original functions.

The binding activity of anti-albumin nanobody 3005Hz6 in the fusion protein to HSA was detected. The detection method was the same as that in "2. Activity assay of humanized antibody" in Example 4. The ELISA results are shown in FIG. 10, and the EC50 of the ELISA experiment is shown in Table 21.

**Table 21. Summary of EC50 of fusion protein or 3005Hz6 binding to HSA**

| Name of protein | EC50 (nM) for binding to HSA |
|---|---|
| V1SA-3005Hz6 | 0.32 |
| 3005Hz6-V1SA | 0.33 |
| V1DP-3005Hz6 | 0.39 |
| 3005Hz6 | 0.25 |

It can be seen from Table 21 that the binding activity of the fusion protein to HSA was similar to that of 3005Hz6 alone, indicating that 3005Hz6 fused with other antibody fragments did not affect the activity of 3005Hz6. Moreover, the bioactive effector molecule could be located at the N or C terminus of the anti-albumin antibody, and different positions basically did not affect the activity of the fusion protein binding to albumin.

### 2. Detection of activity of anti-VEGFA nanobody in fusion protein

VEGFR2 competitive ELISA method was used for the detection. The effects of the fusion protein on the VEGFR2 competitive activity of the fusion protein were compared when the fusion protein bound to or did not bind to HSA.

In the competitive ELISA experiment, a microplate was coated with goat anti-human IgG Fc protein (purchased from Solarbio, Cat. No.: SPA105) at a coating concentration of 5 µg/mL overnight at 4 °C. After the plate was washed with PBST and blocked with milk powder, the VEGFR2 extracellular region-human IgG1 Fc fusion protein (VEGFR2-Fc, prepared in-house) was added. The fusion proteins at gradient concentrations were co-incubated with constant 1 µM HSA or PBST for 30 min, and subsequently incubated with biotin-modified VEGFA165-Avi-His at a constant final concentration (0.4 nM) for 1 h. VEGFA165-Avi-His was prepared in-house by adding a linker, Avi tag, and 6xHis tag to the C terminus of human VEGFA1 65. The VEGFA165-Avi-His expression plasmid and the BirA enzyme expression plasmid were co-transfected into 293-F cells. The protein was biotin-modified on the Avi tag during the expression process with the catalysis of BirA enzyme. The amino acid sequence of VEGFA165-Avi-His is:

APMAEGGGQNHHEVVKFMDVYQRSYCHPIETLVDIFQEYPDEIEYIFKPSCVPLMRCGGCCNDEGLECVPTEESNI
TMQIMRIKPHQGQHIGEMSFLQHNKCECRPKKDRARQENPCGPCSERRKHLFVQDPQTCKCSCKNTDSRCKARQL
ELNERTCRCDKPRRGSGSGLNDIFEAQKIEWHEGGGGSHHHHHH (SEQ ID NO: 39).

The amino acid sequence of VEGFR2-Fc is:

ASVGLPSVSLDLPRLSIQKDILTIKANTTLQITCRGQRDLDWLWPNNQSGSEQRVEVTECSDGLFCKTLTIPKVIGND
TGAYKCFYRETDLASVIYVYVQDYRSPFIASVSDQHGVVYITENKNKTVVIPCLGSISNLNVSLCARYPEKRFVPDG
NRISWDSKKGFTIPSYMISYAGMVFCEAKINDESYQSIMYIVVVVGYRIYDVVLSPSHGIELSVGEKLVLNCTARTEL
NVGIDFNWEYPSSKHQHKKLVNRDLKTQSGSEMKKFLSTLTIDGVTRSDQGLYTCAASSGLMTKKNSTFVRVHEK
PFVAFGSGMESLVEATVGERVRIPAKYLGYPPPEIKWYKNGIPLESNHTIKAGHVLTIMEVSERDTGNYTVILTNPISK
EKQSHVVSLVVYVPPQIGEKSLISPVDSYQYGTTQTLTCTVYAIPPPHHIHWYWQLEEECANEPSQAVSVTNPYPCE
EWRSVEDFQGGNKIEVNKNQFALIEGKNKTVSTLVIQAANVSALYKCEAVNKVGRGERVISFHVTRGPEITLQPDM
QPTEQESVSLWCTADRSTFENLTWYKLGPQPLPIHVGELPTPVCKNLDTLWKLNATMFSNSTNDILIMELKNASLQ
DQGDYVCLAQDRKTKKRHCVVRQLTVLERVAPTITGNLENQTTSIGESIEVSCTASGNPPPQIMWFKDNETLVEDSG
IVLKDGNRNLTIRRVRKEDEGLYTCQACSVLGCAKVEAFFIIEGAQEKTNLEPPCPAPELLGGPSVFLFPPKPKDTLM
ISRTPEVTCVVVDVSHEDPEVKFNWYVDGVEVHNAKTKPREEQYNSTYRVVSVLTVLHQDWLNGKEYKCKVSNK
ALPAPIEKTISKAKGQPREPQVYTLPPSRDELTKNQVSLTCLVKGFYPSDIAVEWESNGQPENNYKTTPPVLDSDGSF
FLYSKLTVDKSRWQQGNVFSCSVMHEALHNHYTQKSLSLSPGK (SEQ ID NO: 43)

The mixture of the fusion protein and VEGFA165-Avi-His was added to the microplate that had adsorbed VEGFR2-Fc, and the plate was incubated at 37 °C for 1 h. The biotin signal of VEGFA165-Avi-His was detected using HRP-labeled streptavidin antibody (Streptavidin-HRP, purchased from Sangon Biotech, Cat. No.: D111054-0001). The plate was incubated at room temperature for 45 min and washed. Subsequently, TMB chromogenic solution was added to the microplate at 100 µL/well for color development for 15 min; a stopping solution was added at 50 µL/well, and the microplate was placed in a microplate reader to read the absorbance at 450 nm (OD450).

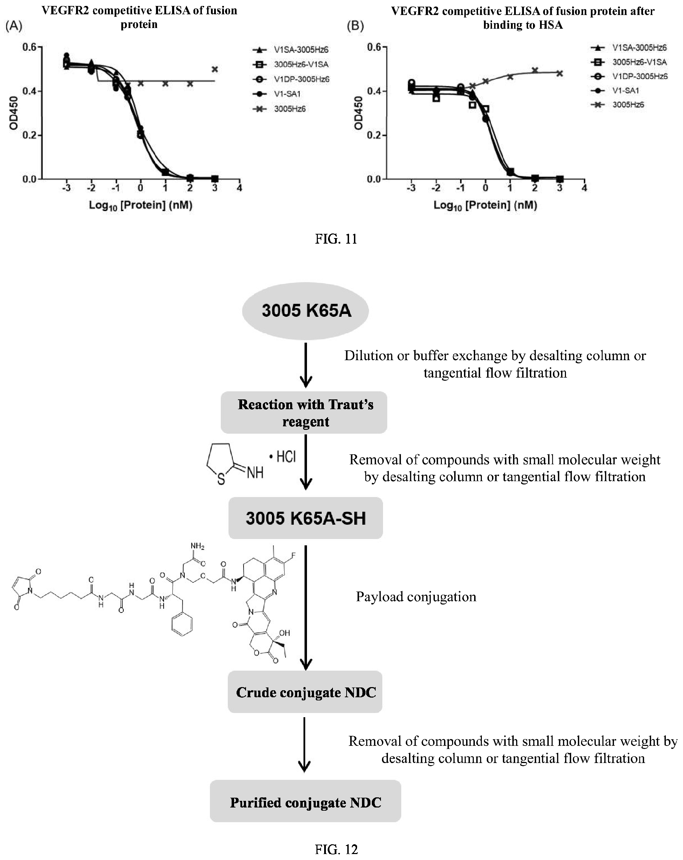

The activity of the fusion protein and the monovalent nanobody V1-SA1 competing for VEGFR2 without pre-incubation with HSA is shown in FIG. 11 (A), and the competitive activity for VEGFR2 after pre-incubation with HSA is shown in FIG. 11 (B). The IC50 of the VEGFR2 competition experiment is summarized in Table 22.

**Table 22. Summary of IC50 of fusion protein and monovalent nanobody V1-SA1 competing for VEGFR2**

| Name of protein | IC50 (nM) of VEGFR2 competitive ELISA without pre-incubation with HSA | Ratio of IC50 of fusion protein to 1C50 of V1-SA1 without pre-incubation with HSA | IC50 (nM) of VEGFR2 competitive ELISA with pre-incubation with HSA | Ratio of IC50 of fusion protein to IC50 of V1-SA1 with pre-incubation with HSA |
|---|---|---|---|---|
| V1SA-3005Hz6 | 0.84 | 1.08 | 1.59 | 0.90 |
| 3005Hz6-V1SA | 0.75 | 0.96 | 2.47 | 1.40 |
| V1DP-3005Hz6 | 0.63 | 0.81 | 1.55 | 0.88 |
| V1-SA1 | 0.78 | 1.00 | 1.76 | 1.00 |
| 3005Hz6 | No activity | No data | No activity | No data |

It can be seen from Table 22 that the VEGFR2 competitive activity of the three fusion proteins formed by the tandem connection of 3005Hz6 and V1-SA1 or V1-DP was similar to that of the monovalent nanobody V1-SA1 without incubation with HSA (the IC50 ratio was 0.81-1.08), indicating that the anti-VEGFA antibody did not affect the anti-VEGFA activity after fusion with 3005Hz6. In addition, the bioactive effector molecule could be located at the N or C terminus of the anti-albumin antibody, and different positions basically did not affect the activity of the bioactive effector molecule in the fusion protein.

To simulate the anti-VEGFA activity of antibodies or fusion proteins in the presence of albumin *in vivo,* HSA was preincubated with the fusion proteins or the monovalent nanobody V1-SA1 in the competitive ELISA experimental system to detect the activity of competing with VEGFR2 for binding to VEGFA. Table 22 showed that under the condition of pre-incubation with HSA, the IC50 value of the monovalent nanobody V1-SA1 was about 2 times that in the experimental system without HSA, indicating that HSA would have a systemic impact on the IC50 value of the competitive ELISA experiment, and the ratio of the IC50 value of the fusion protein to the IC50 value of V1-SA1 was 0.88-1.40, indicating that the VEGFR2 competitive activity of the fusion protein was similar to that of the monovalent nanobody V1-SA1.

The data in Table 21 and Table 22 showed that the fusion protein of the monovalent anti-VEGFA nanobody (V1-SA1 or V1-DP) and the anti-albumin nanobody (3005Hz6) had strong activity in competing for VEGFR2 and binding to HSA. This indicated that in the fusion protein, the antibody elements binding to different antigens could function independently, and the interference between the antibody elements was low. Therefore, antibodies binding to other antigens can be expressed in fusion with the anti-albumin humanized antibody 3005Hz6 to construct a multi-specific and multi-functional fusion protein.

### Example 7: Chemical Conjugation of Anti-Albumin Nanobody 3005 Mutant to Small Molecule Toxin Deruxtecan and Determination of Conjugate Activity

### 1 Construction, expression, and purification of anti-albumin nanobody 3005 mutant suitable for conjugation reaction

For the anti-albumin nanobody 3005, amino acid 65 in its amino acid sequence is a highly reactive lysine residue K located in the CDR-H2 region. Covalent conjugation of K65 located at CDR-H2 with small molecules may significantly reduce the affinity of the conjugate for the antigen albumin. Therefore, in this example, the K65 in the sequence of the nanobody 3005 was modified by point mutation. The lysine residue with a primary amino group was mutated to an inactive amino acid such as alanine A, arginine R, and histidine H. The newly constructed mutants were named 3005 K65A, 3005 K65R, 3005 K65H, etc.

**Table 23. Amino acid sequences of CDRs of antibody 3005 mutants**

| Candidate antibody number | SEQ ID NO: x | CDR-H1 | SEQ ID NO: x | CDR-H2 | SEQ ID NO: x | CDR-H3 |
|---|---|---|---|---|---|---|
| 3005 K65A | 2 | NYYMS | 31 | GISVDGSFLDYADAVAG | 9 | ASGPQ GLRLG AP |
| 3005 K65R | 2 | NYYMS | 32 | GISVDGSFLDYADAVRG | 9 | ASGPQ GLRLG AP |
| 3005 K65H | 2 | NYYMS | 33 | GISVDGSFLDYADAVHG | 9 | ASGPQ GLRLG AP |

**Table 24. Amino acid sequences and nucleotide sequences of antibody 3005 mutants**

| SEQ ID NO: x | Name of antibody | Antibody amino acid sequence |
|---|---|---|
| 34 | 3005 K65A | QVQLVESGGGLAQPGGSLRLSCAASGFTFSNYYMSWARQAPGKGLEWVSGISVDGS FLDYADAVAGRFTISRDNAKNTVYLQMNSLKPEDTALYYCARASGPQGLRLGAPRGQ GTQVTVSS |
| 35 | 3005 K65R | QVQLVESGGGLAQPGGSLRLSCAASGFTFSNYYMSWARQAPGKGLEWVSGISVDGS FLDYADAVRGRFTISRDNAKNTVYLQMNSLKPEDTALYYCARASGPQGLRLGAPRGQ GTQVTVSS |
| 36 | 3005 K65H | QVQLVESGGGLAQPGGSLRLSCAASGFTFSNYYMSWARQAPGKGLEWVSGISVDGS FLDYADAVGGRFTISRDNAKNTVYLQMNSLKPEDTALYYCARASGPQGLRLGAPRGQ GTQVTVSS |

| SEQ ID NO: x | Name of antibody | Antibody nucleotide sequence |
|---|---|---|
| 79 | 3005 K65A | CAAGTGCAGCTCGTGGAAAGTGGTGGAGGTCTGGCCCAGCCTGGAGGATCTCTGC GGCTGTCTTGTGCTGCCAGCGGATTTACCTTCAGCAACTACTATATGTCTTGGGCCA GACAGGCCCCTGGTAAAGGCCTGGAGTGGGTGTCCGGTATCTCTGTGGACGGTAG CTTCCTGGACTACGCCGACGCCGTGGCCGGCAGATTCACAATCAGTAGAGACAAT GCCAAGAACACCGTGTACCTGCAGATGAACAGCCTGAAGCCAGAAGATACAGCCC TGTACTACTGCGCCAGAGCCAGCGGACCTCAGGGACTGCGCCTGGGTGCTCCTAG AGGCCAGGGCACCCAGGTCACCGTGTCTTCT |
| 80 | 3005 K65R | CAAGTGCAGCTCGTGGAAAGTGGTGGAGGTCTGGCCCAGCCTGGAGGATCTCTGC GGCTGTCTTGTGCTGCCAGCGGATTTACCTTCAGCAACTACTATATGTCTTGGGCCA GACAGGCCCCTGGTAAAGGCCTGGAGTGGGTGTCCGGTATCTCTGTGGACGGTAG CTTCCTGGACTACGCCGACGCCGTGAGAGGCAGATTCACAATCAGTAGAGACAAT GCCAAGAACACCGTGTACCTGCAGATGAACAGCCTGAAGCCAGAAGATACAGCCC TGTACTACTGCGCCAGAGCCAGCGGACCTCAGGGACTGCGCCTGGGTGCTCCTAG AGGCCAGGGCACCCAGGTCACCGTGTCTTCT |
| 81 | 3005 K65H | CAAGTGCAGCTCGTGGAAAGTGGTGGAGGTCTGGCCCAGCCTGGAGGATCTCTGC GGCTGTCTTGTGCTGCCAGCGGATTTACCTTCAGCAACTACTATATGTCTTGGGCCA GACAGGCCCCTGGTAAAGGCCTGGAGTGGGTGTCCGGTATCTCTGTGGACGGTAG CTTCCTGGACTACGCCGACGCCGTGCACGGCAGATTCACAATCAGTAGAGACAAT GCCAAGAACACCGTGTACCTGCAGATGAACAGCCTGAAGCCAGAAGATACAGCCC TGTACTACTGCGCCAGAGCCAGCGGACCTCAGGGACTGCGCCTGGGTGCTCCTAG AGGCCAGGGCACCCAGGTCACCGTGTCTTCT |

The amino acid sequences of CDRs of the anti-albumin nanobody 3005 mutants are shown in Table 23, and the amino acid sequences and nucleotide sequences of the mutants are shown in Table 24. The expression and purification methods of the mutants are consistent with "1. Expression of anti-albumin candidate antibody" in Example 3 of the present patent. Briefly, the secretion signal peptide (SEQ ID NO: 37) was added to the N terminus of the mutant protein of antibody 3005, and the linker GGGGS (SEQ ID NO: 41) and 6xHis were added to the C terminus of the antibody. The coding DNA sequence was obtained by gene synthesis, and linked to the expression vector pCDNA3.1(+) through the enzyme cutting sites NheI and XhoI. The plasmids were sequenced successfully, and large-scale endotoxin-free extraction was performed. The suspended 293F cells were used for transient expression, and Ni column affinity purification was performed from the cell culture supernatant. After purification, the molecular weight of the three mutant proteins detected by Q-tof was 13.8 kDa. Bio-layer interferometry (BLI) was used to assay the affinity of antibody 3005 and its mutant proteins 3005 K65A, 3005 K65R, and 3005 K65H for albumin of different species. NTA sensor was used to bind to the antibody (with a concentration of 5 µg/mL) with the His tag. The antigens were separately human serum albumin HSA (Baxter AG), mouse albumin MSA (purchased from Equitech-bio, Cat. No.: MSA62-1000), and rat albumin (purchased from abcam, Cat. No.: ab198656). In the BLI experiment, 8 concentration gradients of the antigens were set to 200, 100, 50, 25, 12.5, 6.25, and 3.125 nM. The antigen and the antibody were bound for 180 s, and dissociated for 360 s.

The BLI detection results are shown in Table 25. The results show that the affinity of the mutant proteins 3005 K65A, 3005 K65R, and 3005 K65H binding to albumin of different species was similar to that of antibody 3005.

**Table 25. Binding rate (ka), dissociation rate (kd), and equilibrium dissociation constant (K_{D}) of antibody 3005 and its mutant proteins 3005 K65A, 3005 K65R, and 3005 K65H binding to albumin of different species**

| Antigen | Antibody | K_{D} (M) | ka (1/Ms) | kd (1/s) | Full R^2 |
|---|---|---|---|---|---|
| HSA | 3005 | 5.38E-09 | 1.31E+05 | 7.06E-04 | 0.9997 |
| | 3005 K65A | 4.67E-09 | 1.34E+05 | 6.26E-04 | 0.9997 |
| | 3005 K65R | 5.45E-09 | 1.39E+05 | 7.59E-04 | 0.9996 |
| | 3005 K65H | 5.44E-09 | 1.19E+05 | 6.45E-04 | 0.9995 |
| MSA | 3005 | 9.45E-09 | 1.83E+05 | 1.73E-03 | 0.9939 |
| | 3005 K65A | 8.87E-09 | 1.66E+05 | 1.47E-03 | 0.9977 |
| | 3005 K65R | 8.15E-09 | 2.12E+05 | 1.73E-03 | 0.9941 |
| | 3005 K65H | 1.04E-08 | 1.54E+05 | 1.60E-03 | 0.9979 |
| Rat albumin | 3005 | 7.79E-09 | 9.97E+04 | 7.77E-04 | 0.9981 |
| | 3005 K65A | 3.00E-09 | 1.26E+05 | 3.79E-04 | 0.9993 |
| | 3005 K65R | 8.20E-09 | 1.24E+05 | 1.02E-03 | 0.9989 |
| | 3005 K65H | 3.53E-09 | 1.13E+05 | 3.99E-04 | 0.9991 |

### 2 Preparation method for 3005 K65A and deruxtecan conjugate (nanobody drug conjugate, NDC)

In this example, the mutant 3005 K65A was used to illustrate the preparation, purification, and activity measurement of the conjugate. The 3005 K65A can be obtained by using methods well known to those skilled in the art to obtain a nanobody intermediate having a sulfhydryl group (3005 K65A-SH), such as: producing a sulfhydrylated intermediate by the reaction of the primary amino group of lysine residue with 2-iminothiolane hydrochloride (Traut's Reagent); producing a highly active sulfhydrylated intermediate by the covalent conjugation of the primary amino group of lysine residue with N-succinimidyl S-acetylthioalkanoate (N-succinimidyl S-acetylthioglycolate, SATA)-like linker and hydroxylamine release; obtaining a sulfhydrylated intermediate by the reduction of disulfide bonds with reducing agents dithiothreitol (DTT), 2-mercaptoethanol, tris(2-carboxyethyl)phosphine hydrochloride (TCEP), etc. after the action of lysine residue with N-succinimidyl 3-(2-pyridyldithio)propionate (SPDP); or reducing the disulfide bonds within the nanobody molecule with a certain concentration of reducing agents dithiothreitol (DTT), 2-mercaptoethanol, and tris(2-carboxyethyl)phosphine hydrochloride (TCEP) to produce a sulfhydryl group; and so on, but not limited to these methods.

The sulfhydrylated nanobody reactive intermediate was covalently conjugated with deruxtecan through a thioether bond to obtain a nanobody deruxtecan conjugate. In this example, the reaction between the primary amino group of lysine residue in the 3005 K65A framework and the 2-iminothiolane hydrochloride at pH 7-10 to form an amidine bond and generate a free sulfhydryl group was described by taking the primary amino group and 2-iminothiolane hydrochloride as an example. The free sulfhydryl group on the 3005 K65A-SH framework of the sulfhydrylated nanobody intermediate and the maleimide group of deruxtecan were subjected to Michael addition reaction to generate the structurally stable 3005 K65A-deruxtecan conjugate (NDC).

The conjugation and purification flow chart of the nanobody-deruxtecan conjugate is shown in FIG. 12.

### 3 Purification and structural characterization of 3005 K65A deruxtecan conjugate

The preparation process of the 3005 K65A-deruxtecan conjugate was as follows: In a buffer containing the sulfhydrylated nanobody intermediate 3005 K65A-SH, a solution of deruxtecan was added dropwise, and the mixture was shaken continuously for reaction. Sodium phosphate salt, sodium acetate solution, or sodium borate can be used as the reaction buffer for 3005 K65A-SH and deruxtecan. The pH at the time of reaction was typically between 5 and 9, preferably around pH=7. The organic co-solvent in which deruxtecan was dissolved may be selected from organic solvents such as N,N-dimethylacetamide (DMAC), dimethyl sulfoxide (DMSO), dimethylformamide (DMF), and N-methyl-2-pyridinone (NMP). In this example, a DMAC solution in which deruxtecan was dissolved was added to a buffer of 3005 K65A-SH containing a sulfhydryl group according to 8-15% v/v, and the mixture was reacted. The reaction temperature was 10-25 °C, and the reaction time was 1 h. After the reaction was completed, 1-2 molar equivalents of a thiol-containing reagent (cysteine or N-acetyl-L-cysteine (NAC)) was added to the above reaction system, and the mixture was incubated at room temperature for 20 min to inactivate the unreacted deruxtecan, thereby stopping the reaction.

The purification strategy of the conjugate was as follows: Purification was performed using a commercially available HiTrap Desalting column (purchased from GE, Cat. No.: 17140801). The desalting column was equilibrated with 5-10 column volumes of phosphate buffer PBS pH=7.4. The HiTrap Desalting column was filled with an aqueous NDC reaction solution (about 1.5 mL), and elution was performed on a rapid protein purifier (AKTA purifier 100, GE) according to the instructions for use of the desalting column. Unreacted deruxtecan, N-acetyl-L-cysteine (NAC), small molecule organic co-solvent, etc. were removed according to the molecular weight to obtain the 3005 K65A-deruxtecan conjugate solution. SDS-PAGE and other methods were used to determine whether the purified 3005 K65A deruxtecan conjugate was successfully conjugated, and RP-HPLC was used to detect the content of free small molecules, such as deruxtecan, in the purified 3005 K65A deruxtecan conjugate. The content of free small molecules in the purified conjugate should not exceed 1% before subsequent cell activity assay.

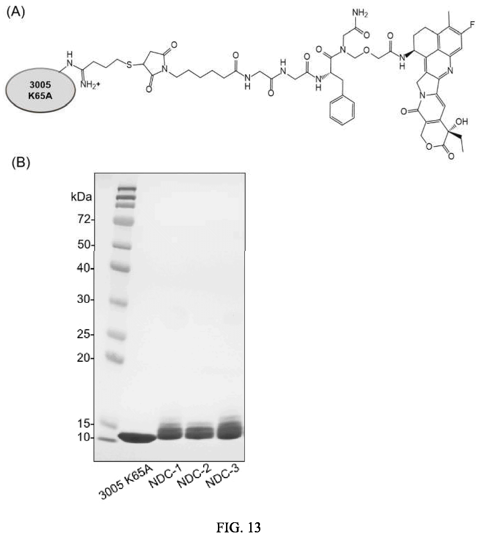

The structure of the 3005 K65A-deruxtecan conjugate (NDC) described in this example is shown in FIG. 13 (A), and FIG. 13 (B) shows the SDS-PAGE gel images of three different 3005 K65A-deruxtecan conjugates (NDC-1, NDC-2, and NDC-3), in which the conjugate labeled as NDC-3 was used as an example for the subsequent ELISA experiment of binding to human serum albumin and the evaluation of the inhibitory activity against the proliferation of pancreatic cancer cell Mia Paca-2.

### 4 Determination of affinity and in vitro activity of 3005 K65A-deruxtecan conjugate

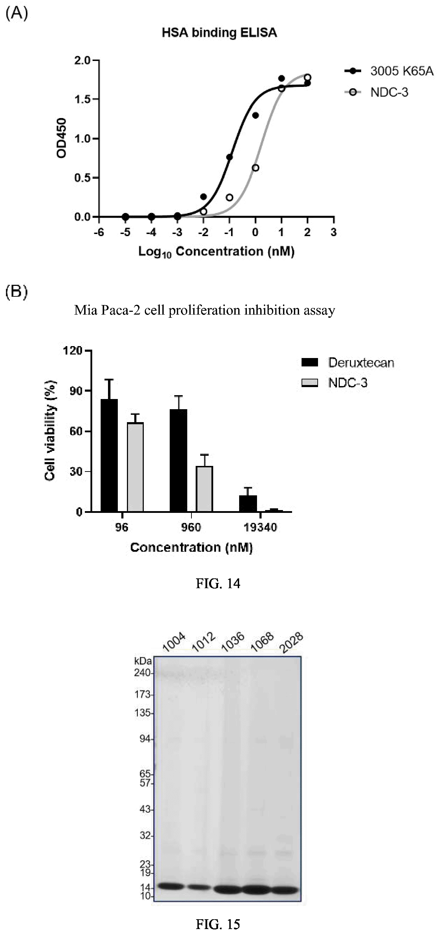

The binding activity of 3005 mutant protein 3005 K65A and 3005 K65A-deruxtecan conjugate (NDC-3) to human serum albumin was detected by ELISA method. In the ELISA experiment, 2 µg/mL human serum albumin was coated, and 3005 mutant protein 3005 K65A and 3005 K65A-deruxtecan conjugate (NDC-3) at gradient concentrations were added. The level of albumin-binding antibodies was detected by HRP-labeled mouse anti-His tag monoclonal antibody (purchased from Proteintech, Cat. No.: HRP-66005). The ELISA experiment results of 3005 mutant protein 3005 K65A and 3005 K65A-deruxtecan conjugate (NDC-3) binding to HSA are shown in FIG. 14 (A). As shown in FIG. 14 (A), after conjugation with the small molecule deruxtecan, the 3005 K65A-deruxtecan conjugate (NDC-3) still had a high binding activity to human serum albumin. The EC50 of the ELISA experiment binding to HSA is summarized in Table 26.

**Table 26. Summary of EC50 values of 3005 mutant protein 3005 K65A and 3005 K65A-deruxtecan conjugate (NDC-3) binding to HSA**

| Name | EC50 (nM) for binding to HSA |
|---|---|
| 3005 K65A | 0.43 |
| 3005 K65A-deruxtecan conjugate (NDC-3) | 1.85 |

The cell proliferation inhibitory effect of the conjugate was evaluated using pancreatic cancer cells Mia Paca-2. The experimental process was briefly described as follows: Mia Paca-2 cells were prepared into 2 × 10⁴ cells/mL with DMEM culture medium (Gibco) containing 10% fetal bovine serum and inoculated into a 384-well cell culture microplate at 25 µL/well. Subsequently, 25 µL of deruxtecan or 3005 K65A-deruxtecan conjugate (NDC-3) diluted with DMEM culture medium at different concentrations was added to each well, so that the final concentrations of deruxtecan or 3005 K65A-deruxtecan conjugate (NDC-3) were 0.96 nM, 9.6 nM, 96 nM, 960 nM, and 19,340 nM. After the addition of the test substance, the cells were cultured at 37 °C with 5% CO₂ for 5 days. After culture, the microplate was taken out from the incubator, 25 µL was removed, an equal amount containing CellTiter-Glo Luminescent Cell Viability Assay (Promega) was added, and the plate was shaken with a mixer at room temperature for 10 min. The luminescence of each sample in the microplate was determined with a microplate reader. The survival rate of tumor cells after 5 days of continuous culture was calculated using the formula: Cell viability (%) = luminescence in the treatment group ÷ luminescence in the control group × 100%

The viability of Mia Paca-2 cells after treatment with the test substance at concentrations of 96 nM, 960 nM, and 19,340 nM is shown in FIG. 14 (B) and Table 27.

**Table 27. Cell viability after treatment of Mia Paca-2 cells with deruxtecan and 3005 K65A-deruxtecan conjugate (NDC-3)**

| Name of test substance | Test substance at 96 nM Viability of Mia Paca-2 cells (%) | Test substance at 960 nM Viability of Mia Paca-2 cells (%) | Test substance at 19340 nM Viability of Mia Paca-2 cells (%) |
|---|---|---|---|
| Deruxtecan | 84.0 ± 14.7 | 76.3 ± 10.1 | 12.4 ± 5.8 |
| 3005 K65A-deruxtecan | 66.6 ± 6.3 | 34.4 ± 8.3 | 1.3 ± 0.8 |
| conjugate (NDC-3) | | | |

It can be seen from FIG. 14 (B) and Table 27 that at the same concentration, the activity of deruxtecan in inhibiting tumor cell proliferation was significantly lower than that of 3005 K65A-deruxtecan conjugate (NDC-3). Therefore, anti-albumin antibody 3005 can be used as a carrier to conjugate with active molecules such as small molecule toxins. The biological activity of the conjugate, such as the inhibition of cell proliferation, is not inferior to or even superior to that of the active molecule itself. The conjugate can potentially improve the cell killing ability of the active molecule or the selectivity of target cells, thereby improving the activity of the small molecule in inhibiting the growth of cancer cells.

### Example 8: Binding Activity of Humanized Anti-Albumin Antibody to Albumin under Different pH Conditions

Albumin is able to bind to the neonatal Fc receptor (FcRn) on the cell surface under weakly acidic conditions. However, its binding capacity is weak under neutral conditions. Therefore, after albumin binds to FcRn, it is endocytosed into the endosome. With the acidification of the endosomal environment, the binding capacity of albumin to FcRn is enhanced, and albumin is brought back to the cell surface by FcRn or is transported across cells. Therefore, albumin can achieve a long half-life by relying on the recycling mechanism of FcRn. Albumin-bound antibodies and fusion proteins thereof undergo similar intracellular transport processes after binding to albumin, so the binding ability of albumin-bound antibodies to albumin under neutral and acidic conditions is critical to achieve long circulation and half-life extension.

The binding rate (ka), dissociation rate (kd), and equilibrium dissociation constant (K_{D}) of humanized antibody 3005Hz6 binding to HSA were detected by SPR method under a near-neutral condition (pH=7.4) and under an acidic condition (pH=5.5, pH of simulated endosomal microenvironment).

In the experiment, the Biacore 8K instrument (Cytiva) was used. The antigen HSA was immobilized using Series S Sensor Chip CM5 and amino coupling. The analyte was 3005Hz6 at gradient concentrations. When the binding and dissociation of HSA and 3005Hz6 were detected under neutral conditions, the components of the mobile phase buffer used were: 10 mM HEPES pH=7.4, 150 mM NaCl, 3 mM EDTA, and 0.05% v/v Tween-20, and the analyte 3005Hz6 was diluted with this mobile phase. When the binding and dissociation of HSA and 3005Hz6 were detected under acidic conditions, the components of the mobile phase buffer used were: 10 mM sodium acetate pH=5.5, 150 mM NaCl, 3 mM EDTA, and 0.05% v/v Tween 20, and the analyte 3005Hz6 was diluted with this mobile phase. In the experiment, the binding time was 150 s, and the dissociation time was 300 s.

The "1:1 binding" model was used to fit the binding rate (ka), dissociation rate (kd), and equilibrium dissociation constant (K_{D}) of 3005Hz6 binding to HSA at pH=7.4 and pH=5.5. The results are shown in Table 28.

**Table 28. Binding rate (ka), dissociation rate (kd), and equilibrium dissociation constant (K_{D}) of humanized antibody 3005Hz6 with HSA at pH=7.4 and pH=5.5**

| pH condition for SPR experiment | Ligand | Analyte Solution | ka (1/Ms) | kd (1/s) | K_{D} (M) |
|---|---|---|---|---|---|
| pH=7.4 | HSA | 3005Hz6 | 5.33E+05 | 1.52E-03 | 2.84E-09 |
| pH=5.5 | HSA | 3005Hz6 | 1.37E+06 | 4.53E-04 | 3.29E-10 |

It can be seen from Table 28 that using the SPR method of amino-coupled HSA, at pH=7.4, the equilibrium dissociation constant K_{D} of 3005Hz6 and HSA was about 2.84 nM; at pH=5.5, the equilibrium dissociation constant K_{D} of 3005Hz6 and HSA was about 0.33 nM, indicating that the humanized antibody 3005Hz6 could strongly bind to HSA under both neutral and acidic conditions, and the affinity of 3005Hz6 to HSA under acidic conditions was 8.6 times stronger than that under neutral conditions. Therefore, it can be inferred that the humanized antibody 3005Hz6 can strongly bind to HSA at physiological pH.

When the complex of 3005Hz6 and HSA together enters the acidic environment of the endosome, the affinity of 3005Hz6 for HSA is enhanced. Therefore, 3005Hz6 can achieve intracellular circulation or transcellular transport by virtue of the binding of HSA to FcRn.

### Example 9: Fusion Expression of 3005Hz6 with Aflibercept and Activity Assay

The humanized anti-albumin antibody 3005Hz6 was expressed in fusion with the marketed drug Aflibercept (fusion protein of VEGFR1 extracellular domain 2 and VEGFR2 extracellular domain 3 with IgG1 Fc). The 3005Hz6 was linked to the N terminus of the Aflibercept protein, and the linker was GGSGGS (SEQ ID NO: 60). The fusion protein was named 3005Hz6-Aflibercept, and the amino acid sequence of the fusion protein 3005Hz6-Aflibercept is:

EVQLVESGGGLVQPGGSLRLSCAASGFTFSNYYMSWARQAPGKGLEWVSGISVDGSFLDYADAVKGRFTISRDNA
KNSLYLQMNSLRPEDTAVYYCARASGPQGLRLGAPRGQGTLVTVSSGGSGGSSDTGRPFVEMYSEIPEIIHMTEGRE
LVIPCRVTSPNITVTLKKFPLDTLIPDGKRIIWDSRKGFIISNATYKEIGLLTCEATVNGHLYKTNYLTHRQTNTIIDVV
LSPSHGIELSVGEKLVLNCTARTELNVGIDFNWEYPSSKHQHKKLVNRDLKTQSGSEMKKFLSTLTIDGVTRSDQGL
YTCAASSGLMTKKNSTFVRVHEKDKTHTCPPCPAPELLGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALPAPIEKTISKAKGQPREPQV
YTLPPSRDELTKNQVSLTCLVKGFYPSDIAVEWESNGQPENNYKTTPPVLDSDGSFFLYSKLTVDKSRWQQGNVFSC
SVMHEALHNHYTQKSLSLSPGK (SEQ ID NO: 61).

The secretion signal peptide IgK (METDTLLLWVLLLWVPGSTG, SEQ ID NO: 42) was added to the N terminus of the fusion protein 3005Hz6-Aflibercept, and the coding DNA sequence of the protein was linked to an expression vector through the enzyme cutting sites HindIII and NotI by gene synthesis and molecular cloning techniques. The plasmids were sequenced successfully, and large-scale endotoxin-free extraction was performed. The suspended 293F cells were used for transient expression, and Protein A affinity purification was performed from the cell culture supernatant to obtain the fusion protein 3005Hz6-Aflibercept.

The nucleotide sequence of the fusion protein 3005Hz6-Aflibercept is:

GAAGTGCAGCTGGTCGAGAGCGGCGGAGGACTGGTGCAACCTGGCGGCAGCCTCAGACTGAGCTGTGCTGCT
TCTGGCTTCACCTTCAGCAACTACTATATGAGCTGGGCCAGACAGGCCCCAGGCAAAGGCCTGGAATGGGTTT
CCGGCATCAGCGTGGACGGCTCTTTCTGGACTACGCCGATGCCGTGAAGGGCAGATTCACAATCTCTCGGGAT
AATGCCAAGAACAGCCTGTACCTGCAGATGAACAGCCTGAGACCTGAGGACACCGCCGTGTACTACTGCGCCC
GGGCCAGCGGCCCCCAGGGCCTGCGGCTGGGAGCCCCTAGAGGCCAGGGTACACTGGTGACCGTGTCCAGCG
GCGGAAGCGGTGGATCTTCTGATACCGGCAGACCTTTCGTGGAAATGTACTCCGAAATTCCTGAGATCATCCAC
ATGACAGAAGGCCGCGAACTGGTGATCCCCTGCAGAGTGACCAGCCCTAACATCACCGTGACACTGAAGAAG
TTCCCTCTGGATACCCTGATCCCTGACGGCAAGCGGATCATCTGGGACTCCCGGAAGGGCTTCATCATCTCCAA
CGCTACCTACAAAGAGATCGGCCTGCTGACATGCGAGGCCACCGTGAACGGCCACCTGTATAAGACTAACTAC
CTGACCCACAGACAGACCAACACCATCATCGACGTGGTGCTGTCTCCATCTCACGGCATCGAGCTGTCCGTGG
GCGAGAAGCTGGTCCTGAATTGCACCGCCAGAACCGAGCTGAACGTGGGAATCGACTTCAACTGGGAGTACC
CCAGCTCCAAGCACCAGCACAAGAAGCTCGTCAATCGGGACCTGAAGACACAATCCGGCTCCGAGATGAAGA
AATTCCTGTCTACCCTGACCATCGATGGCGTGACCAGATCCGACCAGGGCCTGTACACCTGTGCCGCTTCTAGC
GGACTGATGACCAAGAAGAACTCCACCTTTGTGCGGGTGCACGAGAAAGACAAGACCCACACCTGCCCTCCT
TGCCCTGCCCCTGAGCTGCTGGGCGGCCCTAGCGTGTTCCTGTTCCCTCCTAAGCCTAAGGACACCCTGATGAT

CAGCAGAACCCCTGAGGTGACCTGCGTGGTCGTGGACGTGAGCCACGAGGACCCTGAGGTGAAGTTCAACTG
GTACGTGGACGGCGTGGAGGTGCACAACGCCAAGACCAAGCCTAGAGAGGAGCAGTACAACAGCACCTACA
GAGTGGTGAGCGTGCTGACCGTGCTGCACCAAGACTGGCTGAACGGCAAGGAGTACAAGTGCAAGGTGAGC
AACAAGGCCCTGCCTGCCCCTATCGAGAAGACCATTAGCAAGGCCAAGGGACAGCCTAGAGAGCCTCAAGTG
TACACCCTGCCTCCTAGCAGAGACGAGCTGACCAAGAACCAAGTGAGCCTGACCTGCCTGGTGAAGGGCTTCT
ACCCTAGCGACATCGCCGTGGAGTGGGAGAGCAACGGACAGCCTGAGAACAACTACAAGACCACCCCTCCTG
TGCTGGACAGCGACGGCAGCTTCTTCCTGTACAGCAAGCTGACCGTGGACAAGAGCAGATGGCAGCAAGGCA
ACGTGTTCAGCTGCAGCGTGATGCACGAGGCCCTGCACAACCACTACACACAGAAGAGCCTGAGCCTGAGCC
CTGGCAAG (SEQ ID NO: 82)

The binding activity of anti-albumin nanobody 3005Hz6 in the fusion protein 3005Hz6-Aflibercept to HSA was detected by ELISA method. In the experiment, human serum albumin HSA (Baxter AG) was coated at a final concentration of 2 µg/mL at 100 µL/well overnight at 4 °C. After the plate was blocked with 5% skimmed milk powder, the gradient dilutions of the fusion protein were added. The plate was incubated at 37 °C for 1 h and then washed. Subsequently, HRP-labeled goat anti-human IgG Fc antibody (purchased from Abbkine, Cat. No.: A21050) was added, and the plate was incubated at room temperature for 45 min. After the plate was washed, 100 µL of TMB (purchased from Tiangen Biotech, Cat. No.: PA107-01) was added to each well for color development at 37 °C for 15 min, followed by the addition of 50 µL of stopping solution. The absorbance at 450 nm (OD450) was measured using a microplate reader. The EC50 of the ELISA binding assay for the fusion protein is shown in Table 29.

The activity of the anti-VEGF protein Aflibercept in the fusion protein 3005Hz6-Aflibercept was detected by VEGFR2 competitive ELISA method, and the control protein was the marketed drug Aflibercept. The experimental method of competitive ELISA was the same as that in "2. Detection of activity of anti-VEGFA nanobody in fusion protein" in Example 6. The IC50 of the competitive ELISA for the fusion protein is shown in Table 29.

**Table 29. Summary of experimental results of activity assay of protein 3005Hz6-Aflibercept**

| Name of protein | HSA Binding ELISA EC50 (nM) | VEGFR2 competitive ELISA IC50 (nM) |
|---|---|---|
| 3005Hz6-Aflibercept | 0.17 | 3.99 |
| Aflibercept | No binding | 4.52 |

It can be seen from Table 29 that the fusion protein 3005Hz6-Aflibercept had a strong HSA binding activity, and its VEGFR2 competitive activity was similar to that of the marketed drug Aflibercept. The results show that the anti-albumin nanobody 3005Hz6 and the anti-VEGF protein Aflibercept in the fusion protein 3005Hz6-Aflibercept did not affect each other's activity, and both could function independently.

### Example 10: Affinity Enhancement Engineering of Humanized Antibody 3005Hz6

For the 3 CDRs of the humanized antibody 3005Hz6, random mutations at a single amino acid site and two adjacent amino acid sites were performed, and the mutated sequences were displayed on the phage surface by phage display technology. By gradually reducing the antigen input amount and species cross-screening in the screening process, candidate sequences of anti-albumin nanobodies that could bind to HSA, cynomolgus monkey serum albumin, MSA, and rat albumin with an affinity higher than that of 3005Hz6 were obtained. Candidate antibodies 1004, 1012, 1036, 1068, and 2028 were finally obtained. The amino acid sequences and nucleotide sequences of the candidate antibodies are shown in Table 30, and the amino acid sequences of their CDRs are shown in Table 31.

**Table 30. Amino acid sequences and nucleotide sequences of candidate antibodies with improved affinity**

| SEQ ID NO: x | Name of candidate antibody | Antibody amino acid sequence |
|---|---|---|
| 44 | 1004 | EVQLVESGGGLVQPGGSLRLSCAASGFTYANYYMSWARQAPGKGLEWVSGISV DGSFLDYADAVKGRFTISRDNAKNSLYLQMNSLRPEDTAVYYCARASGPIWLRL GAPRGQGTLVTVSS |
| 45 | 1012 | EVQLVESGGGLVQPGGSLRLSCAASGFTFSNYYMSWARQAPGKGLEWVSGISVD GWYLDYADAVKGRFTISRDNAKNSLYLQMNSLRPEDTAVYYCARASGPLWLRL GAPRGQGTLVTVSS |
| 46 | 1036 | EVQLVESGGGLVQPGGSLRLSCAASGFTFTEYYMSWARQAPGKGLEWVSGISVD GSFLDYADAVKGRFTISRDNAKNSLYLQMNSLRPEDTAVYYCARASGPLWLRLG APRGQGTLVTVSS |
| 47 | 1068 | EVQLVESGGGLVQPGGSLRLSCAASGFTFREYYMSWARQAPGKGLEWVSGISVD GSFLDYADAVKGRFTISRDNAKNSLYLQMNSLRPEDTAVYYCARASGPQGLRW WAPRGQGTLVTVSS |
| 48 | 2028 | EVQLVESGGGLVQPGGSLRLSCAASGFTFPNYYMSWARQAPGKGLEWVSGISVD MSFLDYADAVKGRFTISRDNAKNSLYLQMNSLRPEDTAVYYCARASGPVGLRLG APRGQGTLVTVSS |

| SEQ ID NO: x | Name of candidate antibody | Antibody nucleotide sequence |
|---|---|---|
| 83 | 1004 | GAGGTGCAGCTGGTTGAATCTGGCGGAGGATTGGTTCAGCCTGGCGGCTCTC TGAGACTGTCTTGTGCCGCTTCTGGCTTCACCTATGCGAACTACTACATGTCCT GGGCCAGACAGGCTCCTGGCAAAGGACTGGAATGGGTGTCCGGAATCTCCGT GGACGGCTCCTTTCTGGATTACGCCGATGCCGTGAAGGGCAGATTCACCATCT CCAGAGACAACGCCAAGAACTCCCTGTACCTGCAGATGAACAGCCTGCGGCC TGAGGATACCGCCGTGTACTACTGTGCTAGAGCCTCCGGACCTATTTGGCTGA GATTGGGTGCTCCTAGAGGCCAGGGAACCCTGGTCACAGTCTCTTCT |
| 84 | 1012 | GAGGTGCAGCTGGTTGAATCTGGCGGAGGATTGGTTCAGCCTGGCGGCTCTC TGAGACTGTCTTGTGCCGCTTCTGGCTTCACCTTCTCCAACTACTACATGTCCT GGGCCAGACAGGCTCCTGGCAAAGGACTGGAATGGGTGTCCGGAATCTCCGT GGACGGCTGGTATCTGGATTACGCCGATGCCGTGAAGGGCAGATTCACCATCT CCAGAGACAACGCCAAGAACTCCCTGTACCTGCAGATGAACAGCCTGCGGCC TGAGGATACCGCCGTGTACTACTGTGCTAGAGCCTCCGGACCTTTGTGGCTGA GATTGGGTGCTCCTAGAGGCCAGGGAACCCTGGTCACAGTCTCTTCT |
| 85 | 1036 | GAGGTGCAGCTGGTTGAATCTGGCGGAGGATTGGTTCAGCCTGGCGGCTCTC TGAGACTGTCTTGTGCCGCTTCTGGCTTCACCTTCACTGAGTACTACATGTCCT GGGCCAGACAGGCTCCTGGCAAAGGACTGGAATGGGTGTCCGGAATCTCCGT GGACGGCTCCTTTCTGGATTACGCCGATGCCGTGAAGGGCAGATTCACCATCT CCAGAGACAACGCCAAGAACTCCCTGTACCTGCAGATGAACAGCCTGCGGCC TGAGGATACCGCCGTGTACTACTGTGCTAGAGCCTCCGGACCTTTGTGGCTGA GATTGGGTGCTCCTAGAGGCCAGGGAACCCTGGTCACAGTCTCTTCT |
| 86 | 1068 | GAGGTGCAGCTGGTTGAATCTGGCGGAGGATTGGTTCAGCCTGGCGGCTCTC TGAGACTGTCTTGTGCCGCTTCTGGCTTCACCTTCCGGGAGTACTACATGTCC TGGGCCAGACAGGCTCCTGGCAAAGGACTGGAATGGGTGTCCGGAATCTCCG TGGACGGCTCCTTTCTGGATTACGCCGATGCCGTGAAGGGCAGATTCACCATC TCCAGAGACAACGCCAAGAACTCCCTGTACCTGCAGATGAACAGCCTGCGGC CTGAGGATACCGCCGTGTACTACTGTGCTAGAGCCTCCGGACCTCAAGGCCTG AGATGGTGGGCTCCTAGAGGCCAGGGAACCCTGGTCACAGTCTCTTCT |
| 87 | 2028 | GAGGTGCAGCTGGTTGAATCTGGCGGAGGATTGGTTCAGCCTGGCGGCTCTC TGAGACTGTCTTGTGCCGCTTCTGGCTTCACCTTCCCGAACTACTACATGTCCT GGGCCAGACAGGCTCCTGGCAAAGGACTGGAATGGGTGTCCGGAATCTCCGT GGACATGTCCTTTCTGGATTACGCCGATGCCGTGAAGGGCAGATTCACCATCT CCAGAGACAACGCCAAGAACTCCCTGTACCTGCAGATGAACAGCCTGCGGCC TGAGGATACCGCCGTGTACTACTGTGCTAGAGCCTCCGGACCTGTGGGCCTGA |
| | | GATTGGGTGCTCCTAGAGGCCAGGGAACCTGGTCACAGTCTCTTCT |

**Table 31. Amino acid sequences of CDR-H1, CDR-H2, and CDR-H3 of candidate antibodies with improved affinity**

| Name of candidate antibody | SEQ ID NO: x | CDR-H1 | SEQ ID NO: x | CDR-H2 | SEQ ID NO: x | CDR-H3 |
|---|---|---|---|---|---|---|
| 1004 | 2 | NYYMS | 5 | GISVDGSFLDYADAVKG | 52 | ASGPIWLRLGAP |
| 1012 | 2 | NYYMS | 50 | GISVDGWYLDYADAVKG | 53 | ASGPLWLRLGAP |
| 1036 | 49 | EYYMS | 5 | GISVDGSFLDYADAVKG | 53 | ASGPLWLRLGAP |
| 1068 | 49 | EYYMS | 5 | GISVDGSFLDYADAVKG | 54 | ASGPQGLRWWAP |
| 2028 | 2 | NYYMS | 51 | GISVDMSFLDYADAVKG | 55 | ASGPVGLRLGAP |

### Example 11: ELISA Binding Activity Assay of Candidate Antibodies with Improved Affinity to Albumin

tPA secretion peptide was added to the N terminus of the candidate antibodies with improved affinity (antibodies 1004, 1012, 1036, 1068, and 2028 shown in Table 30), and the 6xHis tag was added to the C terminus. Their expression and purification were the same as the experimental procedures of "1. Expression of anti-albumin candidate antibody" in Example 3.

The results of the Coomassie staining after purification of the candidate antibodies with improved affinity are shown in FIG. 15, and the molecular weight of the candidate antibodies was about 14.7 kDa.

The activity of the candidate antibodies with improved affinity binding to albumin from different species was detected by ELISA method.

In the ELISA experiment, the candidate antibody (at a concentration of 5 µg/mL) was coated overnight at 4 °C. Biotin-modified antigen HSA (with Avi and 6xHis tags and the amino acid sequence:
DAHKSEVAHRFKDLGEENFKALVLIAFAQYLQQCPFEDHVKLVNEVTEFAKTCVADESAENCDKSLHTLFGDKLCT VATLRETYGEMADCCAKQEPERNECFLQHKDDNPNLPRLVRPEVDVMCTAFHDNEETFLKKYLYEIARRHPYFYA PELLFFAKRYKAAFTECCQAADKAACLLPKLDELRDEGKASSAKQRLKCASLQKFGERAFKAWAVARLSQRFPKA EFAEVSKLVTDLTKVHTECCHGDLLECADDRADLAKYICENQDSISSKLKECCEKPLLEKSHCIAEVENDEMPADLP SLAADFVESKDVCKNYAEAKDVFLGMFLYEYARRHPDYSVVLLLRLAKTYETTLEKCCAAADPHECYAKVFDEF KPLVEEPQNLIKQNCELFEQLGEYKFQNALLVRYTKKVPQVSTPTLVEVSRNLGKVGSKCCKHPEAKRMPCAEDY LSVVLNQLCVLHEKTPVSDRVTKCCTESLVNRRPCFSALEVDETYVPKEFNAETFTFHADICTLSEKERQIKKQTAL VELVKHKPKATKEQLKAVMDDFAAFVEKCCKADDKETCFAEEGKKLVAASQAALGLGSGSGLNDIFEAQKIEWHE GGGGSHHHHHH, SEQ ID NO: 56) was diluted with PBST solutions with different pH (pH=7.4 or 6.0), and added to the microplate. The plate was incubated at 37 °C for 1 h, and separately washed with PBST solutions with different pH (pH=7.4 or 6.0). HRP-labeled streptavidin antibody (Streptavidin-HRP, purchased from Sangon Biotech, Cat. No.: D111054-0001) was added. The plate was incubated at room temperature for 45 min and washed. Subsequently, 100 µL of TMB (purchased from Tiangen Biotech, Cat. No.: PA107-01) was added to each well for color development at 37 °C for 15 min, followed by the addition of 50 µL of stopping solution. The absorbance at 450 nm (OD450) was measured using a microplate reader.

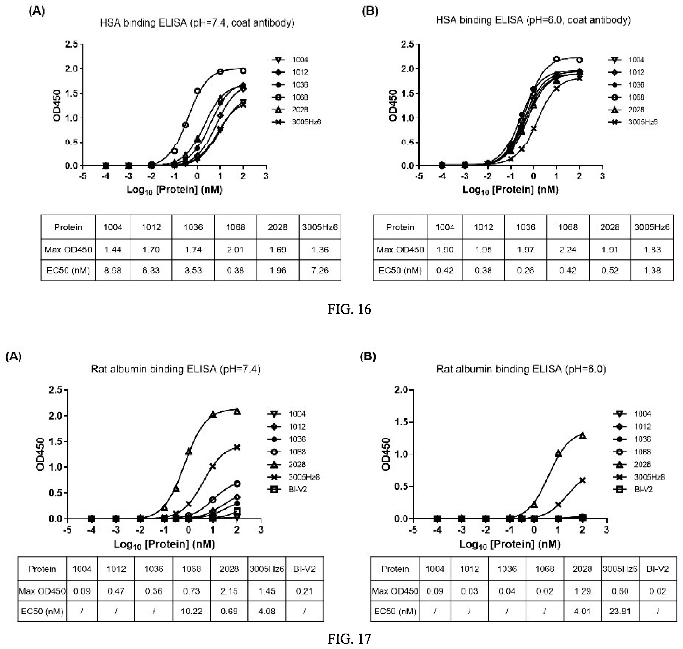

The ELISA experimental results of HSA binding of the candidate antibodies with improved affinity at pH=7.4 or pH=6.0 are shown in FIG. 16 (A) and FIG. 16 (B), respectively. It can be seen that at pH=7.4, the affinity of all candidate antibodies except 1004 binding to HSA was higher than that of 3005Hz6, and the affinity improvement of antibody 1068 was the most significant. At pH=6.0, the affinity of the 5 candidate antibodies binding to HSA was all higher than that of 3005Hz6.

The activity of the candidate antibodies with improved affinity binding to rat albumin was detected by antigen-coated ELISA method. In the experiment, unlabeled rat albumin was coated at a concentration of 5 µg/mL overnight at 4 °C. The candidate antibodies (3005Hz6, 1004, 1012, 1036, 1068, and 2028) were diluted with PBST solutions with different pH (pH=7.4 or 6.0), and added to the microplate. The plate was incubated at 37 °C for 1 h, and separately washed with PBST solutions with different pH (pH=7.4 or 6.0). Anti-His tag monoclonal antibody (purchased from Proteintech, Cat. No.: HRP-66005) was added. The plate was incubated at room temperature for 45 min and washed. Subsequently, 100 µL of TMB (purchased from Tiangen Biotech, Cat. No.: PA107-01) was added to each well for color development at 37 °C for 15 min, followed by the addition of 50 µL of stopping solution. The OD450 was measured using a microplate reader. The control group BI-V2 is SEQ ID NO: 15 selected from the patent US 2023/0050615 A1, and the amino acid sequence is:

EVQLVESGGGLVQAGGSLRLSCAASGLTFSSYAMGWFRQAPGKERERVVSISRGGGYTYYADSVKGRFTISRDNAE
NTVYLQMNSLKPEDTAVYYCAAARYWATGSEYEFDYWGQGTLVTVSS (SEQ ID NO: 57).

The ELISA experimental results of rat albumin binding of the candidate antibodies with improved affinity at pH=7.4 or pH=6.0 are shown in FIG. 17 (A) and FIG. 17 (B), respectively. It can be seen that at pH=7.4 or pH=6.0, the affinity of antibody 2028 for rat albumin was higher than that of 3005Hz6, while the control protein BI-V2 hardly bound to rat albumin.

### Example 12: Affinity Assay of Candidate Antibodies with Improved Affinity for HSA (SPR)

The affinity of the candidate antibodies with improved affinity for HSA was determined by two SPR methods.

In the first SPR method, the instrument Biacore T200 (Cytiva) was used. The anti-His tag antibody was immobilized using amino coupling on the surface of the CM5 chip to capture the candidate antibody with the His tag. The concentration of the candidate antibody was 20 nM, the buffer was 10 mM HEPES pH=7.4, 150 mM NaCl, and 0.05% v/v Tween-20, the flow rate was 30 µL/min, and the capture time was 30 s. The chip that captured the antibody subsequently bound to and dissociated from HSA at different concentrations. The highest concentration of HSA was 37.5 nM or 18.75 nM. The dilution was performed at a 2-fold concentration gradient. The binding time to the chip was 180 s, and the dissociation time was 300 s. The binding and dissociation curves were fitted to obtain the equilibrium dissociation constant K_{D}, as shown in Table 32.

**Table 32. Binding rate (ka), dissociation rate (kd), and equilibrium dissociation constant (K_{D}) of candidate antibodies 1004, 1012, 1036, 1068, and 2028 with improved affinity, and 3005Hz6 with HSA**

| Ligand | Analyte Solution | ka (1/Ms) | kd (1/s) | K_{D} (M) |
|---|---|---|---|---|
| 1004 | HSA | 4.41E+05 | 1.63E-03 | 3.71E-09 |
| 1012 | HSA | 4.00E+05 | 1.41E-03 | 3.52E-09 |
| 1036 | HSA | 4.34E+05 | 1.88E-03 | 4.34E-09 |
| 1068 | HSA | 4.48E+05 | 9.91E-04 | 2.21E-09 |
| 2028 | HSA | 2.39E+05 | 1.31E-03 | 5.49E-09 |
| 3005Hz6 | HSA | 3.94E+05 | 2.93E-03 | 7.42E-09 |

It can be seen from Table 32 that the affinity of the candidate antibodies 1004, 1012, 1036, 1068, and 2028 with improved affinity for HSA was all higher than that of 3005Hz6, and the antibody 1068 had the highest affinity for HSA.

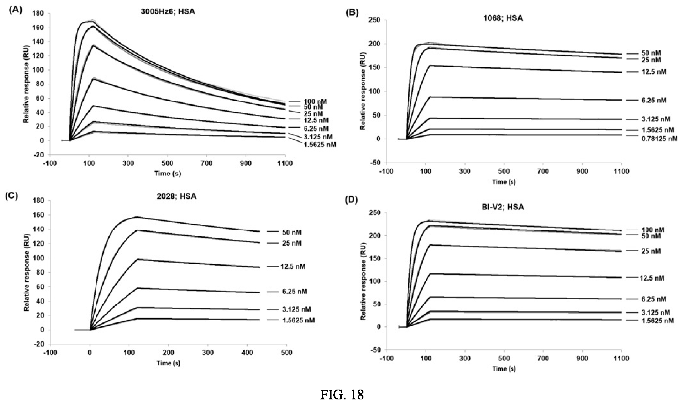

In the second SPR method, the Biacore 8K instrument (Cytiva) was used. The antigen HSA was immobilized using Series S Sensor Chip CM5 and amino coupling (response value: about 1250 RU). The analytes were 3005Hz6, 1068, 2028, and the control protein BI-V2 at gradient concentrations. The buffer containing the protein was 10 mM HEPES pH=7.4, 150 mM NaCl, 3 mM EDTA, and 0.05% v/v Tween-20. In the SPR experiment, the binding time was 120 s, and the dissociation time was 1000 s (for antibody 2028, the dissociation time was 300 s). The binding and dissociation curves of the antibodies 3005Hz6, 1068, and 2028, and the control protein BI-V2 binding to HSA are shown in FIG. 18 (A), FIG. 18 (B), FIG. 18 (C), and FIG. 18 (D), respectively.

The "1:1 binding" model was used to fit the binding rate (ka), dissociation rate (kd), and equilibrium dissociation constant (K_{D}) of the antibodies binding to HSA. The results are shown in Table 33.

**Table 33. Binding rate (ka), dissociation rate (kd), and equilibrium dissociation constant (K_{D}) of antibodies 3005Hz6, 1068, and 2028, and control protein BI-V2 with HSA**

| Ligand | Analyte Solution | Ka (1/Ms) | kd (1/s) | K_{D} (M) |
|---|---|---|---|---|
| HSA | 3005Hz6 | 7.78E+05 | 1.57E-03 | 2.02E-09 |
| HSA | 1068 | 2.29E+06 | 1.29E-04 | 5.66E-11 |
| HSA | 2028 | 8.59E+05 | 4.67E-04 | 5.43E-10 |
| HSA | BI-V2 | 6.17E+05 | 9.64E-05 | 1.56E-10 |

The results show that the affinity of antibody 1068 for HSA was very high, which was about 56.6 pM, and the affinity was significantly higher than that of 3005Hz6 (about 35.7-fold increase). Moreover, the affinity was higher than that of the control antibody BI-V2 (about 2.8-fold increase). The affinity of antibody 2028 for HSA was higher than that of 3005Hz6 (about 3.7-fold increase).

The preferred embodiments of the present disclosure are described in detail above. However, the present disclosure is not limited to the specific details in the embodiments described above. Within the scope of the technical concept of the present disclosure, various simple modifications can be made to the technical solutions of the present disclosure, and all such simple modifications will fall within the protection scope of the present disclosure.

In addition, it should be noted that the various specific technical features described in the specific embodiments described above can be combined in any suitable manner provided that no contradiction arises. In order to avoid unnecessary repetition, various possible combinations will not be illustrated separately in the present disclosure.

## Claims

1. An anti-albumin antibody or an antigen-binding fragment thereof, comprising CDR-H1, CDR-H2, and CDR-H3 of a heavy chain variable region, wherein
an amino acid sequence of CDR-H1 comprises SEQ ID NO: 1 or 58, or an amino acid sequence having at least 80% identity to the amino acid sequence set forth in SEQ ID NO: 1 or 58;
an amino acid sequence of CDR-H2 comprises any one of SEQ ID NOs: 3-4, 6, and 40, or an amino acid sequence having at least 80% identity to the amino acid sequence set forth in any one of SEQ ID NOs: 3-4, 6, and 40;
an amino acid sequence of CDR-H3 comprises any one of SEQ ID NOs: 7-8 and 59, or an amino acid sequence having at least 80% identity to the amino acid sequence set forth in any one of SEQ ID NOs: 7-8 and 59.

2. The anti-albumin antibody or the antigen-binding fragment thereof according to claim 1, wherein X₁ in SEQ ID NO: 58 (X₁YYMS) represents N or E, corresponding to SEQ ID NO: 2 or 49, respectively;
X₂X₃ in SEQ ID NO: 40 (GISVX₂X₃X₄X₅LDYADAVX₆G) represents DS, DA, EG, DM, or DG, X₄X₅ represents SF or WY, and X₆ represents K, A, R, or H; preferably,
X₂X₃ represents DG, X₄X₅ represents SF, and X₆ represents K (SEQ ID NO: 5);
X₂X₃ represents DA, X₄X₅ represents SF, and X₆ represents K (SEQ ID NO: 20);
X₂X₃ represents DS, X₄X₅ represents SF, and X₆ represents K (SEQ ID NO: 21);
X₂X₃ represents EG, X₄X₅ represents SF, and X₆ represents K (SEQ ID NO: 22);
X₂X₃ represents DG, X₄X₅ represents SF, and X₆ represents A (SEQ ID NO: 31);
X₂X₃ represents DG, X₄X₅ represents SF, and X₆ represents R (SEQ ID NO: 32);
X₂X₃ represents DG, X₄X₅ represents SF, and X₆ represents H (SEQ ID NO: 33);
X₂X₃ represents DG, X₄X₅ represents WY, and X₆ represents K (SEQ ID NO: 50); or
X₂X₃ represents DM, X₄X₅ represents SF, and X₆ represents K (SEQ ID NO: 51);
or,
X₇X₈ in SEQ ID NO: 59 (ASGPX₇X₈LRX₉X₁₀AP) represents QG, IW, LW, or VG, and X₉X₁₀ represents LG or WW; preferably,
X₇X₈ represents QG, and X₉X₁₀ represents LG (SEQ ID NO: 9);
X₇X₈ represents IW, and X₉X₁₀ represents LG (SEQ ID NO: 52);
X₇X₈ represents LW, and X₉X₁₀ represents LG (SEQ ID NO: 53);
X₇X₈ represents QG, and X₉X₁₀ represents WW (SEQ ID NO: 54); or
X₇X₈ represents VG, and X₉X₁₀ represents LG (SEQ ID NO: 55).

3. The anti-albumin antibody or the antigen-binding fragment thereof according to claim 1 or 2, wherein the amino acid sequences of the CDR-H1, the CDR-H2, and the CDR-H3 comprise any one of the following groups:
A) SEQ ID NO: 1, 3, 7;
B) SEQ ID NO: 1, 4, 8;
C) SEQ ID NO: 2, 5, 9;
D) SEQ ID NO: 2, 6, 9;
E) SEQ ID NO: 2, 20, 9;
F) SEQ ID NO: 2, 21, 9;
G) SEQ ID NO: 2, 22, 9;
H) SEQ ID NO: 2, 31, 9;
I) SEQ ID NO: 2, 32, 9;
J) SEQ ID NO: 2, 33, 9;
K) SEQ ID NO: 2, 5, 52;
L) SEQ ID NO: 2, 50, 53;
M) SEQ ID NO: 49, 5, 53;
N) SEQ ID NO: 49, 5, 54;
O) SEQ ID NO: 2, 51, 55.

4. The anti-albumin antibody or the antigen-binding fragment thereof according to any one of claims 1-3, comprising a humanized sequence, wherein an engineered site of the humanized sequence is located in a non-CDR region, and preferably, the engineered site of the humanized sequence is located in a framework region and/or a constant region of the antibody.

5. The anti-albumin antibody or the antigen-binding fragment thereof according to any one of claims 1-4, wherein the anti-albumin antibody or the antigen-binding fragment thereof comprises a nanobody, a chimeric antibody, a Fab fragment, a Fab' fragment, an Fd fragment, an Fv fragment, a dAb fragment, a F(ab')₂ fragment, a single chain antibody, or a linear antibody.

6. The anti-albumin antibody or the antigen-binding fragment thereof according to any one of claims 1-5, wherein an amino acid sequence of the anti-albumin antibody or the antigen-binding fragment thereof comprises any one of the amino acid sequences of SEQ ID NOs: 10-13, 16-19, 23-25, 34-36, and 44-48, or has at least 80% identity to any one of the amino acid sequences of SEQ ID NOs: 10-13, 16-19, 23-25, 34-36, and 44-48.

7. Use of the anti-albumin antibody or the antigen-binding fragment thereof according to any one of claims 1-6, wherein the use comprises:
A. use in preparing a fusion construct comprising the anti-albumin antibody or the antigen-binding fragment thereof according to any one of claims 1-6 and a bioactive effector molecule linked to the anti-albumin antibody or the antigen-binding fragment thereof;
B. use in screening for a bioactive effector molecule, wherein a candidate bioactive effector molecule is linked to the anti-albumin antibody or the antigen-binding fragment thereof, and the biological activity of the candidate bioactive effector molecule is detected; or
C. use in detecting albumin.

8. A fusion construct, comprising one or more of the anti-albumin antibody or the antigen-binding fragment thereof according to any one of claims 1-6 and/or a bioactive effector molecule, wherein the bioactive effector molecule is linked to the anti-albumin antibody or the antigen-binding fragment thereof, and preferably, the fusion construct comprises multiple anti-albumin antibodies or antigen-binding fragments thereof, which may be the same or different; the fusion construct may also comprise multiple bioactive effector molecules, which may be the same or different.

9. The fusion construct according to claim 8, wherein the bioactive effector molecule comprises but is not limited to a small molecule compound or a macromolecular compound, and preferably, the small molecule compound comprises but is not limited to any small molecule drug, and the macromolecular compound comprises but is not limited to an antibody, a ligand that activates or inhibits a receptor, a ligand of a protein, a bioactive enzyme, a nucleic acid medicament, or a combination thereof;
more preferably, the antibody in the macromolecular compound comprises an antibody or an antigen-binding fragment thereof to other targets different from albumin;
more preferably, the structure of the antibody or the antigen-binding fragment thereof to the other targets is one of or a combination of two or more of a nanobody, a chimeric antibody, a Fab fragment, a Fab' fragment, an Fd fragment, an Fv fragment, a dAb fragment, a F(ab')₂ fragment, a single chain antibody, or a linear antibody.

10. The fusion construct according to claim 9, wherein the other targets are selected from VEGFA, VEGFB, VEGFR, FGF, FGFR, PlGF, PDGF, TGF, Integrin, Integrin receptors, interleukins (e.g., IL-1β, IL-2, IL-3, IL-4, IL-10, IL-12, IL-15, IL-17, IL-23, etc.), interleukin receptors (e.g., IL1R1, IL2Rα, IL3R, IL4Rα, IL10R, IL12R, IL15Rα, IL17R, IL23R, etc.), PCSK9, TNF-α, TNFR, RANKL, GPCR, GLP1R, CD3, CD105, CD19, CD20, CD22, CD25, CD27, CD28, CD30, CD33, CD38, CD40, CD47, CD80, CD86, CD96, CD99, CD111, CD112, CD123, CD133, CD138, CD155, CD171, Claudin 18.2, OX40, ICOS, CTLA4, 4-1BB, TCR, BTLA, TIM-3, LAG3, Galectin-9, PD-L1, PD-L2, PD-1, TIGIT, EGFR, Her2, PSCA, CEA, FAP, EGFRVIII, BCMA, PSMA, CA125, EphA2, C-met, L1CAM, CS1, ROR1, recombinant Mycobacterium tuberculosis fusion protein, NY-ESO-1, MUC1, MUC16, mesothelin, LewisY, GPC3, GD2, EPG, DLL 3, and 5T4.

11. The fusion construct according to claim 10, wherein the anti-albumin antibody or the antigen-binding fragment thereof is directly or indirectly linked to the bioactive effector molecule, and preferably, the indirect linkage can be a linkage via a linker, a functional domain, and/or a linker for conjugation, wherein
the linker is selected from a linker peptide, an oligopeptide, an oligopeptide polymer, a polypeptide, a polypeptide polymer, PEG, a nucleic acid, a polysaccharide, a fatty chain, biotin, streptavidin, and avidin;
the functional domain is one of or a combination of two or more of an Fc fragment, serum albumin, a cytokine, transferrin, and a scaffold protein;
the linker for conjugation comprises a functional group linker, and preferably, the functional group linker comprises sulfhydryl, amino, hydroxy, and/or carboxyl reactive groups;
more preferably, the bioactive effector molecule is directly or indirectly linked to the N terminus, C terminus, and/or internal residues of the anti-albumin antibody or the antigen-binding fragment thereof.

12. A nucleic acid encoding the anti-albumin antibody or the antigen-binding fragment thereof according to any one of claims 1-6 or encoding the fusion construct according to any one of claims 8-11.

13. The nucleic acid according to claim 12, further comprising any nucleotide sequence of SEQ ID NOs: 63-75, 79-81, and 83-87, or a degenerate sequence thereof, or a nucleotide sequence having at least 80% identity to any nucleotide sequence of SEQ ID NOs: 63-75, 79-81, and 83-87 and having a function of encoding an anti-albumin antibody or an antigen-binding fragment thereof.

14. The nucleic acid according to any one of claims 12-13, comprising any nucleotide sequence of SEQ ID NOs: 76-78 and 82, or a degenerate sequence thereof, or a nucleotide sequence having at least 80% identity to any nucleotide sequence of SEQ ID NOs: 76-78 and 82 and having a function of encoding the fusion construct.

15. A vector, comprising the nucleic acid according to any one of claims 12-14.

16. A host cell, comprising the nucleic acid according to any one of claims 12-14 or the vector according to claim 15.

17. A method for preparing the anti-albumin antibody or the antigen-binding fragment thereof according to any one of claims 1-6 or the fusion construct according to any one of claims 8-11, comprising culturing the host cell according to claim 16, and expressing the anti-albumin antibody or the antigen-binding fragment thereof or the fusion construct.

18. Use of the anti-albumin antibody or the antigen-binding fragment thereof according to any one of claims 1-6, the fusion construct according to any one of claims 8-11, the nucleic acid according to any one of claims 12-14, the vector according to claim 15, or the host cell according to claim 16 in preparing a product for treating and/or diagnosing a disease, wherein the anti-albumin antibody or the antigen-binding fragment thereof and/or the bioactive effector molecule have activity for treating and/or diagnosing a disease, and preferably, the anti-albumin antibody or the antigen-binding fragment thereof serves as a carrier in the product for treating and/or diagnosing a disease.

19. A product for treating and/or diagnosing a disease, comprising any one of the following:
A) the anti-albumin antibody or the antigen-binding fragment thereof according to any one of claims 1-6;
B) the fusion construct according to any one of claims 8-11;
C) the nucleic acid according to any one of claims 12-14;
D) the vector according to claim 15; or
F) the host cell according to claim 16, wherein
the anti-albumin antibody or the antigen-binding fragment thereof and/or the bioactive effector molecule have activity for treating and/or diagnosing a disease;
preferably, the anti-albumin antibody or the antigen-binding fragment thereof serves as a carrier in the product for treating and/or diagnosing a disease;
more preferably, the bioactive effector molecule is a medicament that is covalently bound to the anti-albumin antibody or the antigen-binding fragment thereof.

20. A method for detecting albumin, comprising contacting a sample to be tested with the anti-albumin antibody or the antigen-binding fragment thereof according to any one of claims 1-6, and subsequently detecting the content of a complex formed by albumin and the anti-albumin antibody or the antigen-binding fragment thereof, wherein preferably, the albumin is from a mammal, and more preferably, the mammal is from a human, a mouse, or a monkey.
